# EUROPEAN PATENT SPECIFICATION

(11) **EP 3 493 823 B1**
(45) Date of publication and mention of the grant of the patent: **25.12.2024**
(21) Application number: 17752060.8
(22) Date of filing: 03.08.2017
(51) Int. Cl.: A61K 35/747, A61P 27/02, A61K 9/00

(54) **POSTBIOTIC-BASED COMPOSITION FOR TREATMENT OF OCULAR INFLAMMATION**
POSTBIOTIKA-BASIERTE ZUSAMMENSETZUNG ZUR BEHANDLUNG VON AUGENENTZÜNDUNGEN
COMPOSÉ À BASE DE POSTBIOTIQUE POUR LE TRAITEMENT DE L'INFLAMMATION OCULAIRE

(30) Priority: 03.08.2016 IT 201600081929
(43) Date of publication of application: 12.06.2019
(73) Proprietor: Istituto Europeo di Oncologia S.r.l., 20121 Milano (IT)
(72) Inventor: RESCIGNO, Maria, 20139 Milano (MI) (IT); PENNA, Giuseppe, 20139 Milano (MI) (IT)
(74) Representative: Pace Napoleone, Maria
(86) International application number: PCT/EP2017/069681
(87) International publication number: WO 2018/024833

(56) References cited:
- WO-A1-2008/077229
- WO-A2-2007/125566
- WO-A2-2011/009848
- IOVIENO ALFONSO ET AL: "Preliminary evidence of the efficacy of probiotic eye-drop treatment in patients with vernal keratoconjunctivitis.", GRAEFE'S ARCHIVE FOR CLINICAL AND EXPERIMENTAL OPHTHALMOLOGY = ALBRECHT VON GRAEFES ARCHIV FUR KLINISCHE UND EXPERIMENTELLE OPHTHALMOLOGIE MAR 2008, vol. 246, no. 3, March 2008 (2008-03-01), pages 435 - 441, XP002769562, ISSN: 0721-832X
- DATABASE BIOSIS, [online] 1 May 2011 (2011-05-01), CALDER VIRGINIA L ET AL: "Conjunctival Expression of Interleukin-17 Receptors in Chronic Allergic Eye Disease: An Immunoregulatory Role for Interleukin-17 on Conjunctival Epithelial Cells", XP002729474, retrieved from BIOSIS Database accession no. PREV201200524607
- BONINI S ET AL: "Vernal keratoconjunctivitis.", EYE (LONDON, ENGLAND) APR 2004, vol. 18, no. 4, April 2004 (2004-04-01), pages 345 - 351, XP002769563, ISSN: 0950-222X

## Description

### Field of the Invention

The invention refers to a composition comprising a fermented product of Lactobacillus casei or paracasei species, preferably for use in the treatment of eye inflammation syndromes and to a method for obtaining the same.

The invention relates to the use of the supernatant of Lactobacillus Paracasei in the treatment of conjunctivitis, in particular of Vernal keratoconjunctivitis (VKC).

### Background of the Invention

### Conjunctivitis

Conjunctivitis is an inflammation or infection of the eye conjunctiva. It is characterized by conjunctival vessel dilatation, leading to hyperemia and edema of the conjunctiva, usually manifesting with associated discharge (Leibowitz HM, The red eye, N. Engl. J. Med., 2000;343(5):345-351).

Conjunctivitis can differ based on the aetiology. It can be related to viral or bacterial infections, or it can be allergic. Allergic conjunctivitis is by far the most common one throughout the population and it occurs between spring and summer. Viral conjunctivitis is the most common infectious conjunctivitis and occurs primarily in the summer, while bacterial conjunctivitis is most frequent in children and is observed from December through April.

Conjunctivitis can be primary or secondary to a systemic disease. In the first case, topical treatment is to be preferred, while in the second case systemic treatment should be sought. Treatment depends on the cause of the disease and is carried out with antibiotic-based eyedrops, prescribed for bacterial conjunctivitis; anti-histamines, anti-inflammatroy or steroid eyedrops for the treatment of allergic conjunctivitis and no cure is available for non-herpes viral conjunctivitis (JAMA, 2013;310(16):1721-1729, doi:1 0.1 001 /jama.2013.280318).

### Vernal keratoconjunctivitis (VKC)

Vernal keratoconjunctivitis (VKC) is a chronic, bilateral inflammation of the conjunctiva. VKC affects in particular young children (especially boys) from the age of 4 to 20. The incidence of the disease is 1-10 cases in 10.000 individuals, but it has been rising in the last decades. The exact aetiology of the disease is unknown but VKC is present mostly in allergic individuals (with asthma, atopic dermatitis, allergic rhinitis, etc.) and includes eosinophils and mast cell hyperactivity. The pathophysiological feature is a dense mixed cellular infiltrate with sometimes an extremely thickened epithelium. It shows pronounced capillary proliferation, fibrosis and a thickened extracellular matrix.

The clinical picture is characterised by pronounced subjective symptoms and the emergence of giant papillae, mostly on the upper tarsal conjunctiva. Distinction can be made between a limbal form and a palpebral form. Corneal changes are the most threatening complications with development of corneal erosions and shield ulcers. It is a debilitating disease characterized by burning and itching as well as photophobia. Current therapeutic options are aimed to prevent long-term tissue damage caused by chronic inflammation and depend on the severity of the disease. Mild cases are treated with anti-histamines and mast cell stabilizers. Severe cases are treated with steroids either local or systemic and with cyclosporine under strict medical control. Due to the limited clinical benefit and the adverse side effects of these immunosuppressive drugs, especially in the eye where they may cause increased intraocular pressure, cataract formation and glaucoma, these drugs are far from ideal, and a considerable effort is needed to identify alternatives.

Therefore, there is a need for new drugs or compounds capable of either preventing or treating conjunctivitis without causing side effects.

### Allergy, microbiota and dysbiosis

The microbiota is the community of commensal bacteria inhabiting our body (Yatsunenko et al., 2012). It is emerging that we are made of ten times more microbial than mammalian cells which contribute to a hundred times more genes. Hence, the microbiota provides a set of new functions that over the years our organism has learned to exploit (Qin et al., 2010). This enormously enhances the genetic variation among individuals that is provided by the human genome (Li et al., 2008; Mueller et al., 2006; Qin et al., 2010). One important function of the microbiota is the maturation of the immune system and protection against some infectious agents (Hooper et al., 2012; Khoruts et al., 2010; Reid et al., 2011; Swiatczak et al., 2011). It is becoming clear that especially in the early phases of life the microbiota 'educates' our immune system to deal with both innocuous and harmful bacteria and to establish a balance among the two that is characteristic of a healthy gut. Indeed, the microbiota is composed by symbiotic innocuous bacteria and potential pathogens also called pathobionts (Chow et al., 2011).

Recently, it has been shown that several disorders, including allergic rhinitis and atopic dermatitis are associated with a disrupted microbiota, also called dysbiosis (Robles Alonso and Guarner, 2013). An altered microbial community may trigger allergic reactions characterized by an increase in Th2 type of responses, IgEs, eosinophilia or basophilia and mast cell activation (Cahenzli et al., 2013) (Hill et al., 2012). Also the eye mucosa is colonized by the microbiota and it is likely that dysbiosis may be responsible for some allergic manifestations or for susceptibility to viral or bacterial infections leading to conjunctivitis.

### Probiotics

Probiotics are defined as live microorganisms that exert beneficial effects for the host when administered in adequate amounts. However, the administration of probiotics should not be arbitrary as each strain may produce specific metabolites in the fermented product with distinctive immunomodulatory properties (Klaenhammer et al., 2012). For instance, when the activity of several Lactobacilli on the immune system was compared, it was found that each strain has very specific characteristics (Mileti et al., 2009; Tsilingiri et al., 2012; Tsilingiri and Rescigno, 2013). It was thus found that Lactobacillus Plantarum NCIMB8826 has detrimental effects due to its immunostimulatory properties in mice and on healthy human intestinal tissue (Mileti et al., 2009; Tsilingiri et al., 2012), while Lactobacillus rhamnousus GG and L. Paracasei B21060 can be detrimental only on inflamed tissues like those of patients with inflammatory bowel diseases (Tsilingiri et al., 2012).

By contrast, the inventors found that metabolic products of probiotics - that were called postbiotics (Tsilingiri and Rescigno, 2013) - are very safe also on inflamed tissues, presumably because postbiotics lack the microbe associated molecular patterns that may further activate inflamed tissues (Tsilingiri et al., 2012). The use of probiotics (L. acidophilus) in eyedrops has already been proposed by Bonini et al. and has shown some efficacy in controlling symptoms of VKC in 6 out of 7 patients (Iovieno et al., 2008). In this clinical trial, patients were treated with probiotic eyedrops for 4 weeks, while signs and symptoms of VKC were being recorded.

While symptoms were improved both after 2 and 4 weeks, the improvement of clinical signs was evident after 4 weeks. Prolonged use of probiotic-based eyedrops has not been tested, but it cannot be excluded that it might lead to a permanent change in the eye microbiota composition, or on the community of commensal bacteria inhabiting the human body (Yatsunenko et al., 2012), with unpredictable long-term effects on the eye immune homeostasis.

### The supernatant of Lactobacillus paracasei

The putative therapeutic use of strains of Lactobacillus paracasei strain CNCM I-1390 (Budapest Treaty deposit), redeposited on July 26, 2017 according to Budapest Treaty with CNCM (Collection Nationale de Cultures de Microorganismes, Institut Pasteur, Paris FR) rno. I-5220 (hereinafter also named as B21060), in particular of the fermentation supernatant thereof as an anti-inflammatory in intestinal diseases, is described in WO 2011/009848 A2.

### Description of the Invention

The invention is as defined in claims 1-9. It is herein described the use of a fermented product of Lactobacillus species (postbiotic) for the generation of an eyedrop formulation to protect the eye mucosa from infections and allergic reactions and subsequent conjunctivitis.

The advantage of the postbiotic is multiple. First, there is no introduction of live bacteria that are normally colonizing other mucosal sites in the eye. Second, there are no microbial associated molecular patterns (MAMPs) normally associated with the bacterial cell wall such as endotoxins (LPS) or lypoteichoic acid (LTA), that may be potentially harmful. Third, a postbiotic has anti-inflammatory, Th1/Tregulatory cell skewing and barrier-protective properties on epithelial cells, including corneal epithelial cells. Hence, the use according to the invention of a post-biotic eye-drop is safer and probably more effective than a probiotic eyedrop, with the advantage of not affecting the composition of the eye microbiota by introducing species that are not normally present in the eye.

The invention refers to the use of a fermented product that can re-establish the homeostasis of the eye mucosa and protect it from both types of reaction. The fermented product is obtained at a non-canonical temperature of fermentation. The fermented product can on one side skew the T cell response towards a Th1 type of response that can be protective against pathogens and can counteract the pro-allergic Th2 type of response initiated during allergy. On the other hand, the fermented product does not favour the induction of an acute inflammatory response. The fermented product of the invention has immunomodulation activity both on the cells of the Immune system and on epithelial cells and promotes the formation of the epithelial barrier thus protecting the mucosa from external agents and is therefore particularly suitable for the treatment of conjunctivitis, particularly of VKC. The fermented product can be obtained through the fermentation of different substrates, such as for example sodium lactate.

It is herein disclosed a composition comprising a fermented product of Lactobacillus casei or paracasei species, said species being characterized by comprising in their DNA genome DNA sequences essentially identical to SEQ ID No 1 to 5 sequences, proper diluents and/or eccipients. The fermented product herein disclosed is produced by the method as below disclosed.

In a preferred embodiment the Lactobacillus species is Lactobacillus paracasei, preferably a strain characterized by comprising in its DNA genome DNA sequences essentially identical to SEQ ID No 6 to 18 sequences, more preferably the strain deposited according to Budapest Treaty with no. CNCM I-5220.

Preferred uses of the composition of the invention include use in the treatment of eye inflammation syndromes, preferably of conjunctivitis, in particular of Vernal Keratoconjunctivitis (VKC), in human and veterinary medicine.

It is within the object of the invention a composition further comprising adjuvants, and/or other therapeutic agents known to the skilled person for ocular inflammation syndromes.

Pereferably the composition of the invention is in the form of an eye-drop formulation.

Preferably the composition of the invention comprises the fermented product as above defined at 2-40 % volume, preferably 10-25% vol, more preferably 25% vol. It is within the scope of the invention a method for obtaining the fermented product as above defined characterized by:
a) growing an inoculum of Lactobacillus strain as above defined in a suitable culture medium, at a temperature ranging from 4 to 40°C, preferably at about 37°C, to have a biomass and allowing fermentation of said biomass at a temperature ranging from 4 to 40°C, to proceed for 12 to 36 hours, preferably for about 24 hours, to get a fermented biomass, and
b) centrifuging said fermented biomass to get a pellet fermented biomass and first fermented product;
c) incubating said pellet fermented biomass into a minimum solution (saline, phosphate buffer, H₂O, etc.), preferably comprising a lactate salt, and allowing further fermentation at a temperature ranging from 4 to 40°C, for 12 to 36 hours, preferably for about 24 hours, to get a further fermented biomass;
d) separating said further fermented biomass from a second fermented product by centrifugation.

First and/or second fermented product obtainable from step b) or d) respectively are an object of the invention and collectively will be defined as "fermented product". They may be used as active ingredients for the composition and the eye drop formulation, either individually or combined.

It herein disclosed a method for treatment of eye inflammation syndromes, preferably of conjunctivitis, in particular of Vernal Keratoconjunctivitis (VKC), in human and veterinary medicine comprising the administration of the composition of the invention to a subject in the need thereof.

The invention will be illustrated by means of non-limiting examples with reference to following figures.
**Figure 1****:** Mass spectrometry profile of fermented product of Lactobacillus Paracasei strain CNCM I-5220 obtained by SACI technique.
**Figure 2****:** MoDCs were stimulated with LPS (100 ng/ml) and treated with or without fermented product of Lactobacillus Paracasei strain CNCM I-5220 for 24 h. Concentrations of IL-10 and IL-12p70 were determined by ELISA. Each symbol refers to individual donors.
**Figura 3:** MoDCs were stimulated with LPS (100 ng/ml) and treated with or without fermented product of Lactobacillus Paracasei strain CNCM I-5220 for 24 h. Concentrations of di IL-12p70, IL-10, IL-1β, TNF-α, IL-6 and MCP1 were determined by ELISA
**Figure 4****:** MoDCs were stimulated with LPS (100 ng/ml) and treated with Flac or the control containing mannitol and sodium-lactate for 24 h. Concentrations of IL-10 and IL-12p70 as determined by ELISA . p**>0,01 (two-ways ANOVA-Bonferroni)

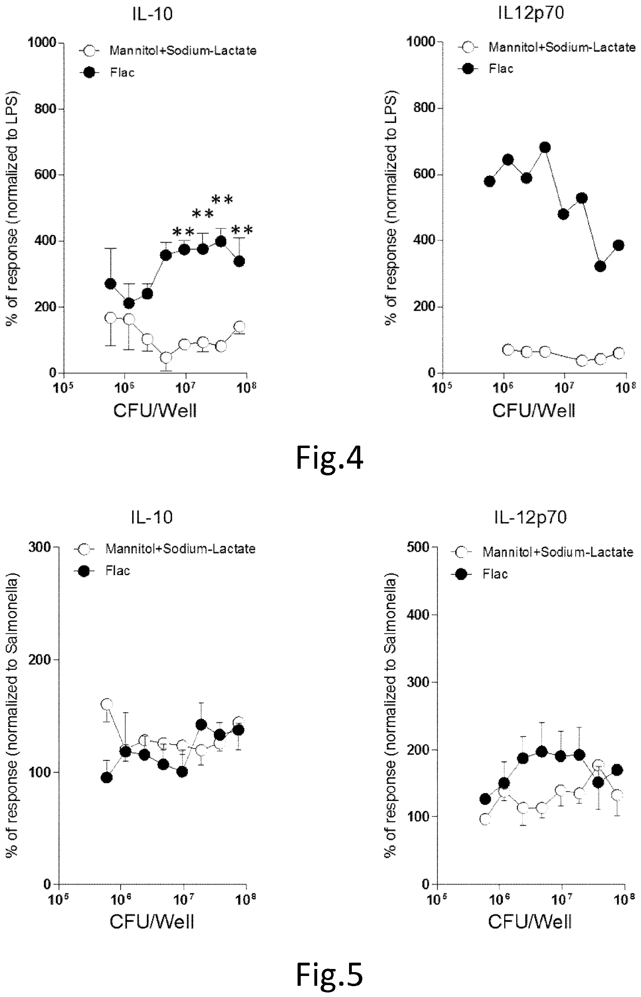

**Figure 4****:** MoDCs were stimulated with LPS (100 ng/ml) and treated with Flac or the control containing mannitol and sodium-lactate for 24 h. Concentrations of IL-10 and IL-12p70 as determined by ELISA . p**>0,01 (two-ways ANOVA-Bonferroni)
**Figure 5**: MoDCs were infected with Salmonella SL1344 (MOI 1:1) and treated with Flac or the control containing mannitol and sodium-lactate for 1 hour. After that, Salmonella was inactivated with gentamicin (100mg/ml) and, after 24 h of incubation, cytokine abundance (IL-10 and IL-12p70) was evaluated in culture medium. Concentrations of cytokines were determined by ELISA.

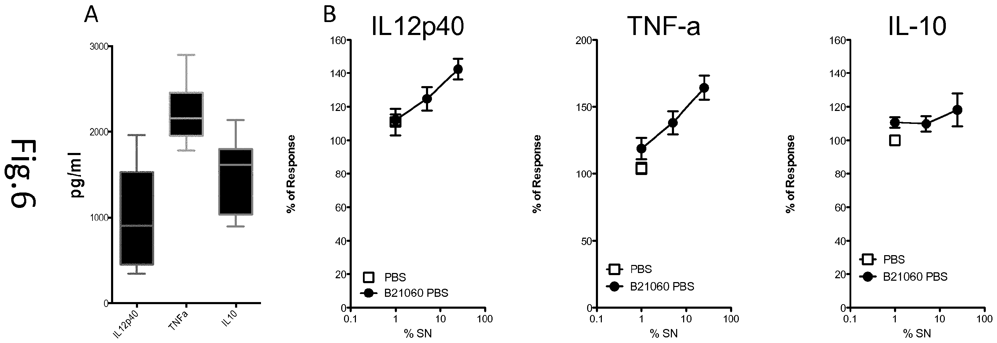

**Figura 6:** PBMCs were stimulated with LPS (100 ng/ml) and treated with fermented product of Lactobacillus Paracasei strain CNCM I-5220 for 24 h. Concentrations of IL-10, IL-12p40 and TNF-α were determined by ELISA. Panel A refers to the absolute concentration of cytokines, while panel B, shows percentage of cytokines refered to LPS-treated only cells.

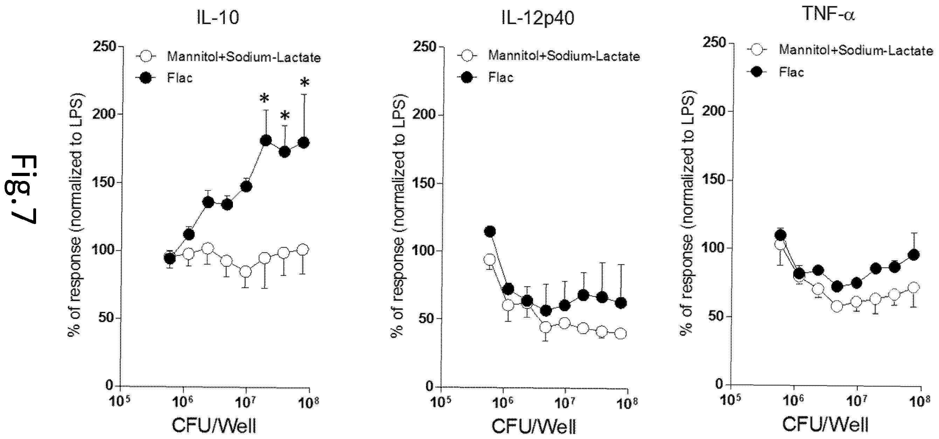

**Figure 7****:** PBMCs were stimulated with LPS (100 ng/ml) and treated with Flac or the control containing mannitol and sodium-lactate for 24 h. Concentrations of IL-10, IL-12p40 and TNF-α were determined by ELISA. p*>0,05 (two-ways ANOVA-Bonferroni)

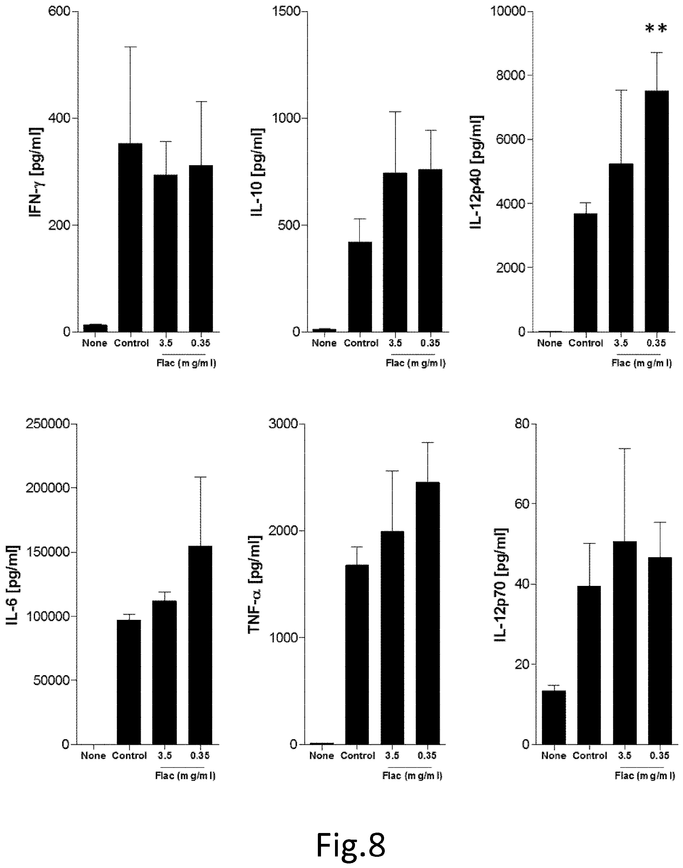

**Figure 8****:** Mice were pre-treated with different doses of Flac (3.5 and 0.35mg/ml) or its control, containing Mannitol-Sodium Lactate, during 4 days. After that, mice were injected intraperitoneally with 200µg of LPS. After 5h, the mice were sacrificed and levels of different cytokines (IFN-γ, IL-10, IL-12p40, IL-6, TNF-α and IL-12p70) were determined by CBA BD Array (BD bioscience). **p<0,01. Mann-Whitney U Test.

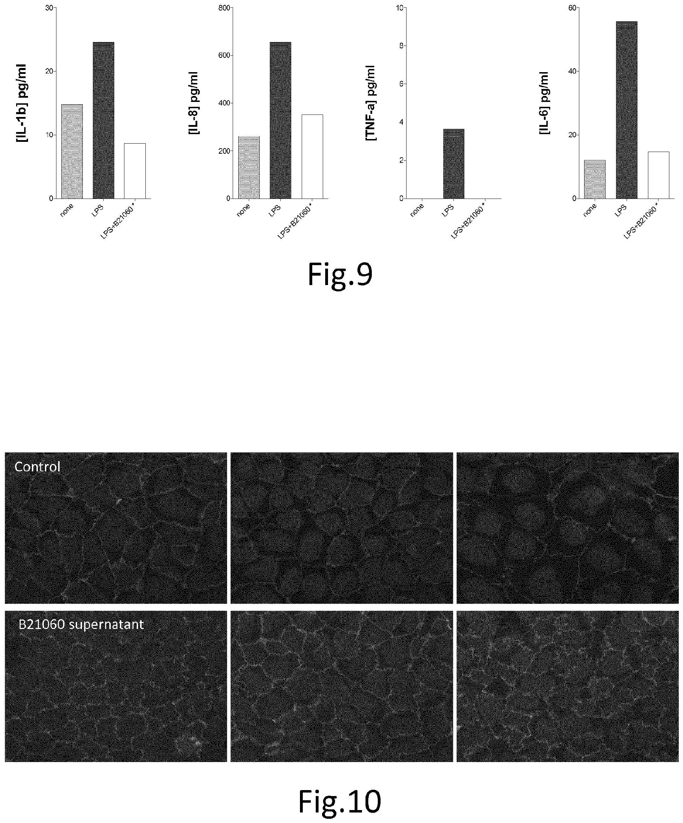

**Figura 9:** Human primary corneal epithelial cels (HCEC, # C0185C Thermo Fisher Scientific) were stimulated for 24 hours with 100 ng/ml of LPS or Staphylococcus aureus Cowanin with or without fermented product of Lactobacillus Paracasei strain CNCM I-5220, Cytokine production (IL-1β, IL-8, TNF-α ed IL-6) was measured by ELISA in cell culture supernatant.
**Figura 10:** ZO-1 expression was evaluated by immunofluorescencence on Caco-2 cells .In the upper row images of cell treated with control; in the lower row cells treated with fermented product of Lactobacillus Paracasei strain CNCM I-5220. Each row shows triplicates of the same treatment.

### Material and Methods

### LC/MS analysis

Mass spectrometry profile of fermented product of *Lactobacillus paracasei* strain CNCM I-5220 was obtained by Surface-activated chemical ionization (SACI) technique (J Mass Spectrom. 2005 Dec;40(12):1550-7.). Fermented product from *Lactobacillus paracasei* strain CNCM I-5220 were treated as follow. Liophilized supernatant were diluted in PBS buffer and 5 µl were loaded on HPLC Ultimate 3000 (Dionex) equipped with Phenomenex Luna C18 (2.0 x 50 mm - particle size 3 µm) column coupled with HCT Ultra (Bruker) spectrophotometer.

### Bacteria culture

*Lactobacillus paracasei* strain CNCM I-5220 was cultured in 3mL of MRS medium anaerobically overnight at 37° C. The following day the culture was restarted (usually at a 1:10 dilution) and the bacteria were harvested and used for stimulation at the exponential growth phase, namely when OD was 0,6 as measured with an Eppendorf biophotometer. *Lactobacillus paracasei* strain CNCM I-5220 supernatants were obtained growing bacteria to OD600 = 0,6 in MRS and suspending biomass in saline solution supplemented or not with sodium lactate (Flac). The resulting medium was then filtered. Fermented product was maintained in liquid form or lyophilized by adding Mannitol.

*Salmonella* serovar *typhimurium,* strain FB62 was grown in 3mL of Luria-Bertani broth and cultured aerobically (in agitation) and used for stimulation at the exponential growth phase, namely when OD was 0,6 as measured with an Eppendorf biophotometer
Monocyte-derived dendritic cell (MoDC) differentiation and stimulation conditions
DCs were derived from human peripheral blood monocytes selected with anti-CD14 antibodies coupled to magnetic beads (Miltenyi, Bologna, Italy). CD14+ cells were incubated for 6 days in complete medium containing granulocyte-macrophage colony stimulating factor (GM-CSF, 5 ng/mL; BD Biosciences) and interleukin-4 (2,5 ng/mL; BD Biosciences) in order to obtain immature MoDCs. MoDCs were incubated with Lipopolysaccharides (LPS) from *Escherichia coli* O111:B4 (Sigma- Aldrich) or Salmonella FB62 (MOI 1:1 bacteria:DC) in the presence or absence of fermented product of *Lactobacillus paracasei* strain CNCM I-5220 for 24 h. Supernatants were tested for cytokine abundance by ELISA (R&D systems) and by cytometric bead assay Flex sets (BD Biosciences).

### Peripheral blood mononuclear cells and stimulation conditions

Buffy coats were obtained from healthy donors having signed an informed consent for research use. Peripheral blood mononuclear cells (PBMC) were separated with Ficoll (GE Healthcare) gradient centrifugation and then resuspended and cultured in RPMI 1640 medium (Lonza) containing 10% fetal bovine serum (Gibco), 1% Glutamine 1% pyruvate, 1% non essential AA and 1% Penicillin-Streptomycin. PBMCs were incubated with Lipopolysaccharides (LPS) from *Escherichia coli* O111:B4 (Sigma- Aldrich) in the presence or absence of fermented product of *Lactobacillus paracasei* strain CNCM I-5220 for 24 h. Supernatants were tested for cytokine abundance by ELISA (R&D systems) and by cytometric bead assay Flex sets (BD Biosciences).

### Human primary corneal epithelial cells and stimulation conditions

Human primary corneal epithelial cells (HCEC, # C0185C Thermo Fisher Scientific) were plated in 96 well plate at 10000 cells/well in the presence or absence of *Lactobacillus paracasei* strain CNCM I-5220 fermented product. After 24 hours cells were stimulated with 100 ng/ml of LPS from *Escherichia coli* O111:B4 (Sigma-Aldrich) or 1:1000 dilution of Staphylococcus aureus Cowan I for additional 24 hours. Cytokine production (IL-1β, IL-8, TNF-α ed IL-6) was measured by ELISA/CBA in cell culture supernatant.

### Caco2 cells culture and stimulation conditions

Caco2 cells were cultured in DMEM supplemented with 10% FBS, 1% Glutamine, 1% nonessential aminoacids, 1% Penicillin-Streptomycin. Cells were seed in Permanox Plastic Nunc^{™} Lab-Tek^{™} Chamber Slide System (ThermoFisher) and stimulated with 100 ng/ml of LPS from *Escherichia coli* O111:B4 (Sigma- Aldrich) in the presence or absence of fermented product of *Lactobacillus paracasei* strain CNCM I-5220. ZO-1 expression was evaluated by immunofluorescencence staining with the following monoclonal antibody: anti ZO-1 clone ZO1-1A12, Invitrogen, at a concentration of 5µg/ml for 1 hour at room temperature. Slices were then incubated with the appropriate fluorophore-conjugated secondary antibody.

Before imaging, nuclei were counterstained with 4',6-diamidin-2-fenilindolo (DAPI).

### LPS-induced endotoxic shock

C57/BL6 mice were purchased from Charles River laboratories. All mice were maintained in microisolator cages in a specific pathogen-free animal facility. All experiments were performed in accordance with the guidelines established in the Principles of Laboratory Animal Care (directive 86/609/EEC) and approved by the Italian Ministry of Health.

Mice were treated orally with Flac (fermented sodium lactate by *Lactobacillus paracasei* strain CNCM I-5220) at 96,72, 24 and 2 hours, before LPS administration (n=5 per group). Control mice received Mannitol-sodium lactate. LPS from *Escherichia coli* O111:B4 (Sigma- Aldrich) was injected intraperitoneally (i.p.) at 200 µg per mouse in 200 µl of injectable water. After 5 hours mice were euthanized by exsanguination under anesthesia and blood was collected. IFN-y, IL-10, IL-12p40, IL-6, TNF-α and IL-12p70 levels were detected in the serum by CBA BD Array (BD bioscience), according to manufacturer's instructions.

### Statistical analysis

Student's paired t test (Mann-Whitney U Test) or ANOVA, was used to determine the statistical significance of the data. Significance was defined as *, P< 0.05; **, P< 0.01, ***, P<0.001. Statistic calculations were performed with GraphPad Prism software.

### Preparation of the L. paracasei B21060 (CNCM I-1390, redeposited as CNCM I-5220) supernatant

An inoculum of L. paracasei B21060 is grown at a temperature of about 37 ° C and is then gently stirred to avoid oxygenation of the culture medium, as MRS medium. The biomass is then allowed to grow for about 12 to 36 hours, preferably for about 24 hours, until the desired concentration of lactobacilli is reached (the preferred concentration corresponds to a microorganism growth equivalent to an absorbance of 0.6 at a wavelength of OD₆₀₀). Then, the culture is centrifuged to separate the bacteria from the culture first fermented product, the former being further processed as below for the preparation of the composition for eye drops of the invention. The first fermented product may be used to get the composition of the invention.

### Preparation of eye drops Formulation A

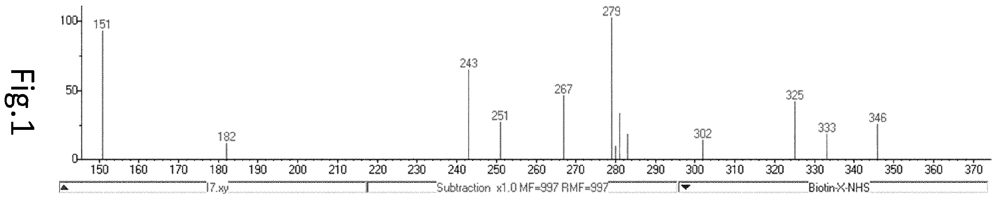

Centrifuged bacteria are transferred to a minimum solution (saline, phosphate buffer, H₂O, etc.) with or without sodium lactate (appr. 5 g/l/) and allowed to ferment for 12 to 36 hours, preferably for about 24 hours without agitation. Then bacteria are centrifuged to separate them from the, second fermented product and the latter is used in the preparation of the composition of the invention. For example the composition is used for eye drop formulation A, and consists of the second fermented product, diluted to 2-40 % vol, preferably 10-25% vol, more preferably 25% vol, in a saline solution or other diluent suitable for the purpose, and properly filtered. The % dilution depends also from the bacteria concentrations obtained according to the above paragraph. Figure 1 shows a mass spectrometric profile obtained by surface-activated chemical ionization (SACI) technique. [Cristoni S, Rubini S, Bernardi LR. Mass Spectrom Rev. 2007 Sep-Oct; 26 (5): 645-56.]

### Stimulation of monocyte derived dendritic cells in the presence or absence of L. paracasei B21060 (CNCM I-5220) fermented product.

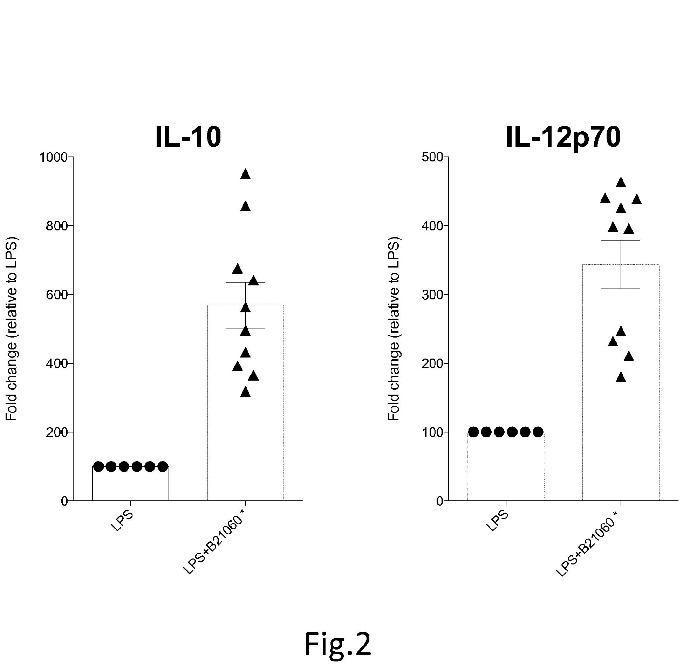

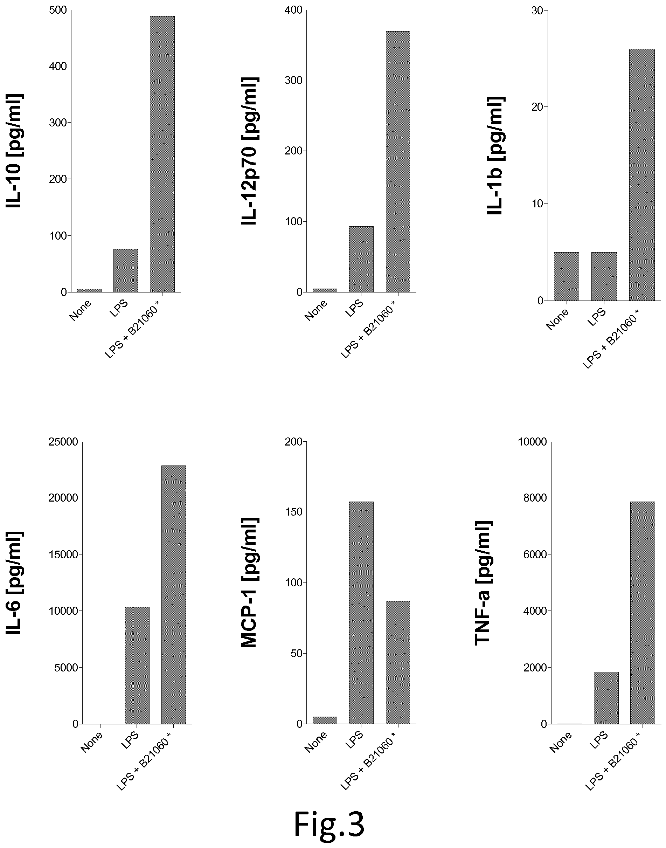

Dendritic cells (DCs) are professional antigen presenting cells involved in the induction of an immune response. According to the type of cytokines that they produce, DCs can skew the T cell response towards different polarizations, including interferon-(IFN)-y producing T helper (Th)1 T cells, interleukin (IL)-4 producing Th2 T cells, IL-10 producing T regulatory cells, or IL-17 producing Th17 T cells. Each one of these cell types is responsible for distinct immune responses. In particular, Th2 T cells are involved in controlling the growth of parasites, Th1 in bacterial and viral infections, while T regulatory cells protect from inflammation and tissue damage. In humans, the most studied DCs are the ones that are generated from monocytes: monocyte derived (Mo)DCs. The activation of MoDCs with bacterial derived lipopolysaccharide drives DCs to produce both inflammatory (IL-12) and anti-inflammatory cytokines. Here it is shown that coincubation of MoDCs with a fermentation product of L. paracasei obtained under a non-canonical temperature amplifies the production of cytokines by MoDCs, including IL-12p70 and IL-10 (Figure 2). It also promotes the increase of IL-1 β, IL-6 and TNF-α, but not MCP-1 (Figure 3). Similar results have been obtained also using as substrate sodium lactate (Fermeted Lactate, Flac) in response to LPS (Fig.4). When Salmonella Thyphimurium was used as stimulus, a slight increase in IL-12p70 was observed and no change in IL-10 production (Fig.5). These results indicate that the L. paracasei fermented product has a strong immune modulatory activity on MoDCs which however is not skewed to either inflammatory or anti-inflammatory response.

### Stimulation of peripheral blood mononuclear cells in the presence or absence of L. paracasei B21060 (CNCM I-5220) fermented product.

Peripheral blood mononuclear cells (PBMC) are a heterogenous cell population that includes myeloid as well as lymphoid immune cells. The amplification of the immune response observed on MoDCs suggests that L. paracasei fermented product may foster the activation of an immune response. Infact, when tested on PBMCs, the L. paracasei fermented product led to increase of IL12p40 cytokines without affecting the already high levels of IL-10 (Figure 6). Similar results have been obtained also using as substrate sodium lactate in response to LPS (Fig.7). Together, these data suggest that the fermented product may activate DCs on one side for a proper activation of an immune response, and mantaining elevated level of IL-10 which protects the host from immunopathology.

### In vivo administration of L. paracasei B21060 (CNCM I-5220) fermented product modulates LPS induced cytokine production.

LPS administration in vivo in mice induces what is called a cytokine storm that can lead to endotoxin shock. When mice were administered first with L. paracasei B21060 fermented product (Flac) and then with LPS we observed an increase in IL-12p40 and IL-10 and, as expected, no change in cytokine IL-6, TNF-α and IFN-γ (Figure 8). This suggests that L. paracasei fermented product does not influence the induction of protective Th1 responses and by increasing IL-10 production it thoese not induce overt inflammation.

### Stimulation of corneal epithelial cells in the presence or absence of L. paracasei B21060 (CNCM I-5220) fermented product.

Corneal epithelial cells protect the inner chamber of the eye from the infection by microorganisms. These cells produce a series of inflammatory cytokines that can mediate the inflammation. L. paracasei B21060 fermented product can inhibit the inflammatory response in corneal epithelial cells in response to LPS. Epithelial cells were stimulated with LPS in the presence or absence of L. paracasei B21060 fermented product and cytokine production was tested. As shown in Figure 9, L. paracasei B21060 fermented product could drastically reduce the production of IL-8, IL-1β, TNF-α, IL-6. This indicates the L. paracasei B21060 fermented product interferes with the production of inflammatory cytokines and counteracts inflammation.

### Analysis of tight junction proteins after stimulation of epithelial cells in the presence or absence of L. paracasei B21060 (CNCM I-5220) fermented product.

Figure 10 shows in the top line the epithelial cell images of three different experiments and treated only with a control solution, while the corresponding inferior line images show the same cells, this time treated with the supernatant of the invention. From the examination of these images we observe that tight junctions are modulated positively (upregulated) as a result of the supernatant of the invention, notably showing greater barrier properties.

Mucosal sites contain highly specialized epithelial cells, that are sealed by the presence of junctional complexes, including tight junction (TJ) and adherens junction (AJ). These form a tight physical barrier that maintains tissue homeostasis and protects from external insults. Alterations of junctional complexes are at the basis of several pathological processes. L. paracasei B21060 fermented product favors TJ formation. Epithelial cells (Caco2) were grown to form TJ in the presence or absence of L. paracasei B21060 fermented product and ZO-1 expression was analyzed. As shown in Figure 10 L. paracasei B21060 fermented product favors TJ formation by increasing ZO-1 expression. This suggests that L. paracasei B21060 fermented product can potentiate the formation of epithelial barrier protecting the tissue from external insults.

### Sequences

In the following are disclosed five gene sequences, representing specific core genes of L. paracasei and L. casei species.
SEQ ID 1 
ATGAATCAAAAAGCTTTGAATCAATTTCCTGAACTTACCTACACAGAACAAGTGTCGGTTGTTGGC
GGCGATTTGTCAGTCGAAGTCATCATGAAAGGTATCTTCACCGGTATCTTTGATGCTGGGTACCA
AGTGGGTCAGTCAATCGCAAAATGGGTTAAGTAA
SEQ ID 2 
ATGAATCAAAAAGCTTTGAATCAATTTCCTGAACTTACCTACACAGAACAAGTGTCGGTTGTTGGC
GGCGATTTGTCAGTCGAAGTCATCATGAAAGGTATCTTCACCGGTATCTTTGATGCTGGGTACCA
AGTGGGTCAGTCAATCGCAAAATGGGTTAAGTAA
SEQ ID 3 
ATGCCCAAAAGGGTCGATCAACATATACGTTCACGCCTTAAAGGCTTTACTTTAATTGAAGTGGT
GGTCAGCCTGATTTTACTTGCGGCGGTCATGCTGTTATGGCGACCGGTTTTATTGCATGTCACGC
GGTTCACGCTTCAAGACCATGTGCTAATCACGTCATTGCAAGCAGAGCATGACTTGCAAATGTTT
GTACGAGATAAAAAGTTGCGGTCTGTGGCCTTAATGTCGGTAAGGGTGAGAAGTCCCGAGAAAG
CTTACACGATCAATTTTTATCAGACCAAACATTTTCGCGGTATGGTTCGTGTGATGGGATCTGAAA
ATGGGCATATGCCATTATTTACGCATCTAACCGGTGTCAATTTTAGCAAGGTAGCTCAAGGCTTT
CGCTATCGTCTGTATTTGACGACTTCGCAGAAGATTGACGGTGGGGTGCAAATCGATGAAGATA
CGCGGTAG
SEQ ID 4 
ATGGCCGCTGATTTCACCCAATTACAACAAGCCATTCGCTTGCTCAATGCCCATACTCGAGCTGC
TGATGAGCAAGCGTGGCAAGTGCTTTTTGATCGTTGGCTGGCAACTTTATCCTCTGAAACTCGCC
GGCAAATGCAAACAGTTCGGTTTAATCATGCCCAATTGACGTTACTCACAACGCTGGATCAAAGC
AGTCGCAAACAACTGCGCAATCAGGATTTAACCGCTGCTGTTCCGTTCTCACAAGGCCTAGTCTC
ACGCTATGTTGCTCGCCTTGTTCAATTAAACTTGCTGACAAAATTATCCTTGCCCGACAATCGCAA
GGCCTACATTGTTGCACTAACTCCGCTTGGTCAACAAGTCGCTGCCTTACATCAGCAAATGCATC
ATCACACAAATGCTCAACTCGCCTCTGTACTTCATACCCTTGATCCACAAGATGTTCAAACTACCA
TTCAGGTACTCACAAAACTGACGGCTCAGCCTTTACATCCCAAGTCTTAG
SEQ ID 5

ATGGGCGGTGTCATTTGTTACGCGGTGCCGGTCTTTTGGAAAAGAATACTTCGCAGACACCTGAT
TCACGAGATTAAGACCCTGAATCAAGGATTGCAGTTATCAAGCAAAGCCATGAGCCAATTAATTG
ATCCGGAAAATCCTTATATGGTATTTGCTGATGAAAATGGTGAACTGGATTTTTCATTTTTGTGGC
TAGGCAACTTGCGTCAATTGAGGCGTGAACTGCGTCTAATTAAAGAACAGAAAGCTAGGGTTTGA

In the following are disclosed unique sequences of L. paracasei B21060 with respect to the L. paracasei species publicly available in NCBI databases. SEQ ID 6 to 8 refers to gene sequences, whereas SEQ ID 9 to 18 refers to genome DNA sequences.
SEQ ID 6: 
ATGAGTAAATATAAAGTTATTATTTGGGGATTAGGAAACGTTGGTCGTTCCGCAGTGAGAATGAT
CGCGGAAAGACAAAATATTTTTGAATTGGTTGCAGCCGTTGACGTTGATCCAAAGAAGTTAGGTA
AGGATGCCGGAGAAGTCTTTGATTTTGACAAAGTCGGCGTCAAAGTTTCAGATGATATTGATGCA
GCCTTGAAACTTCCAGCTGACATTGTGCTCGACTTTTGCCCAACGGAAATGGACAAACAAGGAAC
ATTCATGCCTTCTGCTATTCGACTCGCCAAATCGCTCGATGCCGGTAAAAACGTTATTACCACGA
TTCCGGTATATCATGTTCAAGACAGTCAGCCAGAAGTATATGAATATCTAAATGAACATGCTAAAG
CACATAATGTTGCTTTTGTACCATTTGGACTTTTGCCAGGCGATTATGCCTCATATATCCCACTAG
TTTTGGCCGGGGCCATGGGCCACGTGGATAAAATTGTTGTTCAATCCGGTGAAGATGACTGGCA
CAACACATCAGGCTGGGTCGATGTCTTCTCATATGGCGGCGATATCAATAAATATCCAAAACCAG
ACTCAGACGAAGATCTCTTGGCTAAGTTCATTTATGCTTATTATTCATCCGGCGTATACGAGATGG
CCGATAGGATCGGTCTGAAATATGATACCTTCAAACCAGAGCATGAAGTCTTCACTGCACCCAAA
 
GATTTGGAAACGATCAAGGGTACAGTCAAAAAGGGCAGCATTTATGCCCACAGATTTACCATGGC
ACTTTACAACGGCAACGAACAAGTAGCCGCCTTAAGATATGTTCATAAAGTTGATAATAAAGAGA
CACCAGAATTACCGATCAATAATACGATTCATATTGAAGGCTTGCCGTCAGTCGATGCGCAGATC
GATGGATTGATCCCAGAAAGAGAAGGCTACGTTTCATCAGCCGCTCCAGCAGTCAACTTGATCC
CTAGCATTCTCGAGACCGACAAGACAGGTTATGTTGAAGTCTGCGACCTTCCAGTAGTGATTGCC
AGGCCATTGGATATTGGCGCAAAAAAATTAGTCTAG
SEQ ID 7: 
ATGGCAACCTATTCGCAGATAGAACTAGACATAATCAAATCATTTAAAGGGCTGATGAAAGACCA
TGAATTCACTGAGATCTCAATTAAAATGATCGCTGAAAAAGCCGATATCACTCGACGCGGCTTTTA
CAATCACTTCTTAGATAAATATGATCTTGTCAGTACCATCTTTGAGCATGATCTTTTTCCAACAGTC
ATCAGTTTGACGAATATCAATGACTGGGATCAAGGGTCGCTGTTTATCGTGAATTATCTCCAAGA
CAATCGCGACTACTATAAAAAATTGTTGTCGCTTGAAGGACAAAACTGTTTACAGACAGACTTTTA
TAAATTGACTGAGATGCAGATTGGGATCTTGATCCCAGAAATATTGGTCGGTAGGAAAATTTCTG
ACGAAGATCAGGCATTTTTAAGCGATTATTATTTTCACGCTTATATGGGACTGACTACCGAATGGG
TCAAAGGTAAATATGGTTTTTCAACTCAGGAGTTCGTTAAACGGTGGAAAGCCTTACTCAATAATT
CAATGCATAATTATCTGGACAACTACGCTCGATGA
SEQ ID 8: 
ATGAGTAAATATAAAGTTATTATTTGGGGATTAGGAAACGTTGGTCGTTCCGCAGTGAGAATGAT
CGCGGAAAGACAAAATATTTTTGAATTGGTTGCAGCCGTTGACGTTGATCCAAAGAAGTTAGGTA
AGGATGCCGGAGAAGTCTTTGATTTTGACAAAGTCGGCGTCAAAGTTTCAGATGATATTGATGCA
GCCTTGAAACTTCCAGCTGACATTGTGCTCGACTTTTGCCCAACGGAAATGGACAAACAAGGAAC
ATTCATGCCTTCTGCTATTCGACTCGCCAAATCGCTCGATGCCGGTAAAAACGTTATTACCACGA
TTCCGGTATATCATGTTCAAGACAGTCAGCCAGAAGTATATGAATATCTAAATGAACATGCTAAAG
CACATAATGTTGCTTTTGTACCATTTGGACTTTTGCCAGGCGATTATGCCTCATATATCCCACTAG
TTTTGGCCGGGGCCATGGGCCACGTGGATAAAATTGTTGTTCAATCCGGTGAAGATGACTGGCA
CAACACATCAGGCTGGGTCGATGTCTTCTCATATGGCGGCGATATCAATAAATATCCAAAACCAG
ACTCAGACGAAGATCTCTTGGCTAAGTTCATTTATGCTTATTATTCATCCGGCGTATACGAGATGG
CCGATAGGATCGGTCTGAAATATGATACCTTCAAACCAGAGCATGAAGTCTTCACTGCACCCAAA
GATTTGGAAACGATCAAGGGTACAGTCAAAAAGGGCAGCATTTATGCCCACAGATTTACCATGGC
ACTTTACAACGGCAACGAACAAGTAGCCGCCTTAAGATATGTTCATAAAGTTGATAATAAAGAGA
CACCAGAATTACCGATCAATAATACGATTCATATTGAAGGCTTGCCGTCAGTCGATGCGCAGATC
GATGGATTGATCCCAGAAAGAGAAGGCTACGTTTCATCAGCCGCTCCAGCAGTCAACTTGATCC
CTAGCATTCTCGAGACCGACAAGACAGGTTATGTTGAAGTCTGCGACCTTCCAGTAGTGATTGCC
AGGCCATTGGATATTGGCGCAAAAAAATTAGTCTAG
SEQ ID 9 Position 102558..102986 
AAAAACGGCTTAGAACGCTCATATTTGCGTTCTAAGCCGTTTTTATCAGCATAGGTTCTTGACACC
AATAAACATCTTTAGTAATTGATCAAATTTAGGCAATGTGCTTTTGTCGGTGAATGGCGATAGCCC
TACCGAAGCTTCAGCTGAGGTTCTTCTGAGCCACGCAAGCGAAGCGCGCTAGGGCAAGCCAAC
GGCGCGCAGGCGAAGCCGGAGTTAAATGTGGCGCAGCCACACCTTTTTAGGGAGCAACGCGAC
CAGAATTTTGTATGGGGTTTGGGAAGAGGTTCTCCCCAAGGTCTTTTGTGGTTATTAACAAGCAA
AACACAAACACAAGCCTCGCGCGCGTTATATATACTTCTAAATACTTTTAAATACTTTAAGTACTTA
GGGAGACGAGAATGGCTCAACCACGCGTTTAAATCGACT
SEQ ID 10 Position 103624..103864 
ACGACCTCTCGACCACCCACTGCCTCACCAATCCCCAGGTGAACCGGGCCAAGGGCACTACCG
AGCAACCCGACCCCTATATCCCGGTGGGCGTGGTGAAGCAGACCGATGGGGGCATCGTGGTGC
GGGGCGCGCGGATGCTCTCCACGCTGCCCACGGCGGATGAGCTTTTAGTCTTCCCCAGCACTT
TGCTCAAAGAAGGGCCGGGAGCCGACAAGTACGCCGTGGCCTTCGCCATCCC
SEQ ID 11 Position 254291..261674 
ATTGATCGCCTCCGGGTCACTTATATGTAACTAATAATACTCCCTTCTCTCTTTAGTTACAATAGG
GTACAGCCTATCGAATCACTTACGCTTCCACTTTGAGATAACTTTTCGTTATTATTTATCAACGGC
ATTAACGATATCATTAACTGTTTGCAATGCATCGCTCAGTACACTAATTGGTGCTTGTTCAATATA
CTGCATGTGTCGTTGTACAAAATCAAAGGTGTGAAATTGTAACGGATTCACGTACCCTTCTATTTT
TTCAGTCTGAATCGGTACCATCAAGCCTGTTTCAGCTAAGCGATTATGTTTGGCATGCGTGATCG
GACAAACCAACGCTAATCCAGTCATTTTGGCATATTGTTGATTGCTAATAACGAGCGCTGGTCGT
CTTTTTTGAATTTCATGACCCCGACTTGGCATGAAATCAATACTCACAACATCACCTTTGCGTGGT
TGATAATGCCTAGTCCCACTCACTTGGTAATACCTCGTTTTCTAACGTAATATCTTGTTGGTGTAC
TTGTTGCTTGTACCAATCACCTTCAAATGGATTGCGGTGCTTCGGCAGATAAAGAATGCCACCAT
CATCACGTTGCTCAACTGTAAATTCAGTTCCATCGGCGATTTTAATACTCTTTGGAATGGTTAATG
TAATGGAATTGCCAACCCTTCTTGCTTTAACTGTCATTGAAATCATCCTTTCGTATACACCGAGTA
TACACCAGCGCGAATGAATCTGCAACTCTTGTGCCCTCTTGTGTACAAACACCACTGTCAATTTA
CTTTTGCCTATTGTGCTTTATCTCTTCTCGTTCTGTCATTAGTATGCCACCAACACGGCCGACTTC
ATCCGGCTCACCTTTGATGACGCCTTGAATACCATCGATTTGCTTCCAAGTCTTTACTTCCAAGTT
CTTCAAAGACTTTGATATCGTTTTTCGATGCCGGCAAACACTTCTTCATTCTGATGCCTTTCAAAG
AATTCCTCTAGATCATTCATCAGAGATTCTCCTCACTTAAACCTAGTTGGCGTCAAATTCCATCTC
AGCAATCGAATCTTCTAGGCTGTCCATTACTTCGTATGTTTCAATGAAGTTAACCCAGCTGTCATA
TGGATGTAGTTTGCTTTCTTTAATGCTAGTTCTAAGCTTTTCGTACACGTTGTCAATAAACTGCTCA
ACCATCCTATTAACCGCACTATTAATAACCGCTTCGTTCCACTGCTTAGATGCTGCACGCTTTCGA
TTTTGACTATGCTGATGGGACAGCGCCCGGCTTCTGAACAACGCTACTGCCCTGCTGTTAGCATT
CAGATAGGTTTCAAATTCTTCACGATCCATTACGTTTCCTCCTCAAAATAAGCCTCATTTCATAGC
ACAGCTTCAGCAAAAGGCATGTCATCCTACATGCCTTTTTTCTGTTGCTCTTCAATATCAGTATAA
AACGTCCTGCCGCTTTAGGCAAACGTATGTTCGCTATTAAGAACATACGTTTGTATAATAACTATA
AAAGATTTAAAGGAGGTCAATCGTATGGAAAACAATGTCCCGCGTGAAAAATGGCTTTACCCTGA
CCGCTGCATGAAGAAATGGCTGGGCTGGATTCTAAGCGACCATTCCGCCTATATGGAAGAAGCG
GCTATCTCAGAACAACCGGTGCTCCCAAAGCCTGAACAGACACAAGAAACCATTAATGGCGTACT
CGAAGATGCTTGGCAAAACTCAAAAATTGTCGCAGTTCAAATCGGTACGCCATACGATGATCTTC
TGTTACCGGATATTGAAGGCGCCGTGATTGGTCATTGGGACGCTCAGGTTTATCTACAGCTTAAA
ACTGGTGAGATGCAATCCATTAATGCAGCGGACATTCGCAATGTGCAACTGCTCAATCCAGATCG
GTGGTGGGCGTTAGTATGACGACACCATTAGATGATCCAACAAGGTTACCGGTACACGACATTAT
GTGCATTGACTGTAAGTCCTTTTACGCCTCAGTTGAAGCTATCAGACGCGGGATTCATCCGTTAG
CCGCCGACATTGCTGTTCTCAGCAAAGGTAATTCTCCTGGCGGTTTGGTGCTGGCTGCTAGTCC
CAACTGCAAAAAGCGTTACCACGTAGGACTGAGTACACGCCGTTTTCAGCTAAGGGACGATATG
TAGGTAGAACTTGCTGAACCGCGGATGGCTAATTACATTCGCAAGAATTACGGTATCAATCGTAT
TTACCGTCAGTTTACTGACGATGCTCACTGGTCTCCCTATTCCGTTGACGAGTCCTTTATTGACGT
TACCCACCCCCACAATCTCTTCGGTTCTAATGAAGAAATTGCTACCCAAATACAGAAGAAGGTGT
TTGATCAGTTTGGCATTGTCACAACAGTTGGCATTGGGCAAAATCCCCTATTGGCAAAATTAGCC
CTAGATAACGAGGCTAAGAAGTCAACGCCTTGGCAAGCCACTTGGACTTACGATCGTGTGCCAG
AAACAATATGGAAACTTGATGACTTGGTTGATTTTTGGTCGATTGGTAATCGAACTGCCAAGAAG
CTTAACGCGATTGGCCTTCATAATCTTTACGACTTGGCTCATGCAGACCGCGCCATTCTGCACCA
AAGATTCGGTGTTCTCGGTGATGCCATGTACTTTCACGCATGGGGTATTGATTACTCAGACTTAA
CCCGCCGCTACTTACCACGGGCCGAAAATAAAGGCTACGGCAATAGTCAGGTACTCATGCGTGA
TTACACTCAGGCAAGGGAGATTGAAGTCATGCTTAGCGAGATTGCTGATCAAGTGGCTGGCCGA
ATTCGCCATCACCAAGTCCAAGGTGAGGTCATTAGCGTTGGCATTGGTTATGCTGATGCAGAAGA
AGCTGGCACCTCCGGTTTCGGTGCGCAAATGAAGATTGATCCCACAAATCGCACAGACGATTTA
ATTCGCGCTACTCGATTTCTCTTCCATAGTAAGTGGAACGGACACGCTGTTAGAAATGTCTCAGT
TCGCGTTAATCGCATCAGCCAAGCAAGTACAATGCAACTTAGTCTATTTGAATCAGCAGAGAAGG
AGGAAGCAAACGCGGCTCCTATGCTGTAATTACGGATAAAAGAATCACCATCATTAGGTTTTTCG
TCTAACAATTTTAGGAAACTTCACTTTCTAGGTCGTAACTTTATTTTTGCAATCTAGGGTTTTTTAA
ATATATACATTTTAGCTCGTTTGTGTTTAATATTATAATCACAACTATACCAATGATAAATGTCTAAC
ATAAATATACAAACATGTTGACAGAAGCTCTTGAATACGTTTACAATTATTTCGTTCAGGCGAGCT
TTGTTTTTGAAAAAGTATTAATACAAGATAACTAGGTTAGTGGCTGTTGAATTAGGCCCCCGATTT
CGGGACCACGACAGTCACTTGATACTCGATTTTTATCGTTTGCTGGCTTGATCGTACATTGAACG
 
AAATTGGTACAGAAAAAAGAGCTAAGAGCCGCTCCAAATTAGCCAAAACGATTGCGGCGTCAATG
CTTACGGCGATCGTTTCTGCAGTTTTAGCTGTTACCAGATCAAGTCCTAGTTTCCCTTTGATGAAG
GCAAACTCACGCTCGATCTCACCTCGTCGATTTTCGGCTTGTCGGTCTGCCTTACGTTTGGCCG
GATCGACCTGCTTCGGCCGACGGCCCAATCTAGGACCGCTAAGTTTGATCCCAAGATCTGCGCA
CAGCCCGATATTCGCCCGAGTCCGATAAAGCGTATCAGCCAAGATCTCATCCGGGTATGTACCA
TACGTGTCAAAATAATGGTCGATCGTTGCTGGTAAGTCAGCACTTTCGTTAAACGCATTGAACGC
AAACCGTTCAACGGCCACGACGCCATGACTGATCGATACGTCGATCTTGGGCCCGAATTCGACC
GGATCCTTTGCTTTGCCGCGAATGATCGGTCGGATCGCTGGTTGATCAAGGCTTACGATCCGAT
CCGCGACTCGGTGAGTGTGCTGTCGATACATTTCAGTTTGTTGCTCATACAATTTTCGAATGATC
GTTAATCGTTGTGTCTGCCGTTGATTCAATTGCCCGCCTTGTGCTTGCAGTTCTTTGACGTAACG
CAAGTCACGTCGGATGTACTGTAATTGAGCCTTGATCTGCTTATGGGTCGTTTTCACCCAACGGC
GGGGTTTACGTGAAAAGGCGGTCCACGTTTGGTGGGCCTTGCGCTTATAGGTACGCGGCGGTTT
GACCGCTAATTGCTTGGCCATGGCTGCGATGAATCGCTCTAAATTGAGCCGCGCCTGATTGAGT
AGCTGCGTATCCTGCGGATACTTGATCTTTACTGGGACCGCAGTCGCATCAGTGATCAAGATCTT
CTGATGGCCAAGTTTAGCTTGGAGGCGATCGCGGACAAAATCGCTAATGATGTTCGTGATCAACT
CGGAAAGCGGCGCGATCCGGCGCCTGAAATAGGACAGCACCGAAAATGAAAACGGTGCTTGCG
GCTGATACTCTGGCAGGCCAATAAAATACTGATAAGCCGGTGTATCGCGGATCGCTGCGACTAA
CTCACGGTCCGATAGCTGAGTGCGCTGCTTGATCAGTTGGGCGCCATAAAGCAGCCGAAAGGG
TTTACCTGCCCATCCTAAGTTAGACGGGAAAGCCAATTGGTACGCCTCTTCTAGTTGCGGCCACG
GAACTTGGTCGGCCAGTTGGACCCACTCGTTATCTGGACTTAATGGGGTGCTTAAGCCGCTACC
AAACGATTTGATCGATAATTGAACGGCTTTTCGACGATAAACCATGATCCATGCCTCCGATAGGG
TCGTGTCAAATGCAAACGAAATGAGCACGATCCGTAAATTCATATGCATTCATTATACGACGATAA
CGGGTTCAACTCGCATCAAATGTGGTTATATCAAATTATTCAACAGCCACTAGGTTAAGATCTTCA
TTTAAGTGATATTCATTTGCAAGCAATTGAAAATTACTCATCACGAAGAGGATTTCATTGGCCATA
TTGGATAGCACGCAAATCACTTGCTTTAAGAAAATCAGTTCCTTTAATGAGTCTCTTAAAGGACGG
GGCTCTCACTTGTACTCACAATCAATGTTAACTGGAGATCAACAATATGGTCATAGATTCCCATAA
TAACATTGACTTGACTATCTAAAAGAGGCTTCTAACTTTGATATTGGTGGGGTTATTGGTTGCTTG
GCTGTAAGCAGATAATCTTAACTTGGGTTATTTTCATTGTGTTGTAAAGACATTTGTTATAAAGGC
CGAAGTTATCGCTTTGACTTGTAATAAATTATTTTTGATTGAGATATCAGAAAATAAACGGGGGAT
AATAATGAAAAAGATTATTAGGATTGTTCTTTGTGTTGTTAGTTGCGTTAGTATCATGGTCGGATC
GCTTGGGTTCTATTCAACTCCAAAGATCGTTAAAGCCGACAGTACATCTGTTACGGATGTCGACA
TTAATACCTATATTTCTAGCATGACACTTGATCAAAAAATTGGACAAATGTTTGTAGCACGAACCT
CACAAGATACTGATAAAGCTCGTGCTGATATAGCAAAATATAATCTTGGCGGGCTGATTGTTTATG
GTGTTGATTTCACTAGTGTTAAAGGGACAACAGCTACAGAAGCTCAGAATAACTTCAAGATGAAG
ATGCAAGGCTTTCAAAACTCGGCAAGTCTGCCACTATTGATTGGTGTTGATCAAGAAGGAGGGG
CAGTCTCACGCTTATCACAAAATCCTCTAATTGCCAACGGCAGAAGTTTTCCTTCACCACAAATG
GCTTATGCTAATGGTGGAATGACCAATGTAACAAAAGAAGCTAGTGAAGTCGGAACTATTCTAAA
AAATCTGGGCATTAACTGGAACTATGCACCAGTTGCCGACAGTACGCCTGACACCTCTAGTTTTA
TTTATGGTAGAACCTTTGGTCAAGATTACTTGGCTACTGCAAACTATATTACGAATGTGATCCCTG
CGTGGCAAAATGCTGGCATTGCCGCAACTCTCAAGCATTTCCCTGGTTATGGATCCGCGATTGAT
ACGCATACGGATTTTGCAGTCGTTACAAAGTCTAAGGAGGATTTTGAAAAAGAGGACTTGCTTCC
CTTTAAGTCCGGTATTACAGCAGGGGCAGATTCTGTAATGATTGCACATATAGTAATGCAAGCTG
TTGACCCAGTGTATCCAGCATCATTATCACGGAAGGTCGTTACCGATTTGTTGCGTAATGAACTT
GGGTATAATGGCTTAATAATTACCGATGCATTGGAAATGGGGGCCATCAAGCAATTTGCTCAAGA
ACATGATCAAGTTCCTGTTGATGTTCTTGCTGTTGAAGCAGGGAATGATTGCATCATGAATAACG
ATTATGAAACCGCTATTCCACAGATTCATGCAGCAGTAACTAATGGAACTATTAAGGAATCAGAAA
TCAATGAACACGTTTTCCGTATTCTTGATCTCAAACGCAAATTAGGGTTGTTAACTAAAGGACAAC
TTCAGCAAAAAAAAGTTCAGGTTGACAATGTTTCCTACAGCAGTGACAACAAAAAGGCAACTGTG
AGTGGAACAGTTGTTGATAGTGATTGGCAAGTTGGAGAACCATTATCGGTTAAAGACTCGACTGG
GAAGGTCATTATTACCGCAGACGTTGGTGCCGGTGGTAAGTTTACTTTCGATGTTCCTACTAAGT
CCCAAGAACAAGTATTAACTCTGACTACTAATTTACCCAACATCGCTGATTCTCAAATAACTATTAA
GGCTGTGAGTTCATCGAATACTAACAAAGCTTTGCTAGAAAACTTGATCAACGCTGCTGAACAGT
TGGATAGTAATCAATATACTGTCAAGTCGTGGGAAGAATTACAAACTAAACTAACTGAATCAAAAT
CGATTCTGAACAATGATAGTGCTACACAAGATCAAGTAGACGCTTCCGTTAATGCTCTACAAATTG
CCCTTAAGCAATTAGTTCCTGTATCAAATAGCGGAAATAATGGTCAAAGCTCTAATGATAGCAGTA
ACCAAAGTTCATCTAGCAGTAGTGGCAAAGAATCATCCAGCAATAGCAATGCCAATATTACTAGT
AAGGATCAGTCAGCTAAGGATTCAAATACGAGGCCTAAAGACCATAGTCTTTTGCCAAGTACAGG
TGAACGGGTGATGACGGGAATTTCTGTTCTAGGGGTAATTTTAATAGCTTGTGTGACTATATTATA
TATTCGGAAAAAAGGACGCAGCTTTTAATTAGTCTCTGCGTCAACTGGCGTTAAAAACTAGATTGA
 
AGTAATAAAGTTACCACCTGGAAAGAGGCATGCTCATTGCTTGCAAGGGTGTCGACGTGTAATAG
AAAAGTTGGGG
SEQ ID 12 Position 325750..327159 
TGGCGTGGGCAACGTGCACGTTTTCTAGTCGCTAAACTGTGAACAATGCTCGTGCTAAATGCAAA
ACTGAGCAAGGAGATGAACTATAAGCGGGGGACCCTTTGCTATTGAGGAGGAAGGCGAAGTAGA
GAAAGAGCGGTGATTTGAACTCGAAACAGCGGCGCCGCAGGCTAGCAGCACTGTTAGATTAATC
GCCAGCAGCAGATACTAGACAGCTTCTTAAAGGCTTGATAATAGCGTTGCGCCATTTCAATGGAA
CTAGTGGTCAAAATCGCATTGTAGTTGCCATGACCCAAACTCGTTTTACGCGGGCCTTTTTGTAA
AATATATTGAACAACTTGGTTAATATGTTCATCGGTCTCAAAGTCAGCCGGAGTTAAATACGTTTC
TTCCAAGTCCTTCACTGACATCGCTTGAATCTTGGCTTGAATTTTCGCTTCATCAGCCGTACTCAG
CAGGCGGCCTTGCTTATCACGACGAGCTTTAACGCGCTGAGTTTCTTTTTCAAGGGCCCGGGTT
ATTAAAGCGTCTTTACCAATCGTTGTCACGTGTTCCACATTAAATGGTAACACTGCTTGGTCCTCT
AAGGCGTCTCGCAAGTTATAAACATGACAGACTTTACCAAATAGCTCCTCCGTCGTAACTGCTAG
ATCACCTTTGAGCTGTTTCTTATTTTCATTAAAAATGGGGGTGCCAGTGTAACCATACCAGTTACT
ATTGATAAACGCTGCTCGAATTTCCTTTTGCATCTTACCAAACTGCGACCGGTGGACTTCTTCAAC
AAAGAAGATCACCCGTTGCTTTAAAGTCTTACTAAAGCGGGATTGCTTACCGGTTGCCAGCTGGA
CTTGCGTTTTTTTGACCGCCCGATGGAGCTTTTGAATCGAGGTGACCAAGACCTTACCGTCATTT
TGTTGCAATTTACGCATTAAATCACCGGTGTTTTGGGCTTCGTTAATGGCAATATCATCATTGGCA
GCATAGGCACTAAAGTTGCTGGTTGTCTGTTCGTCTAAATCCCGCCGGTCAACTAAGAAGATGAC
CTTATCGACACCAGGATCTTGCGCAGCTAATTTAGCGGTTTTATATGAGGTGAGTGTTTTACCAG
AACCCGTGGTATGCCAAACGAAACCATCCTGATGGTCATGAATCCGGTGCATCACGGCTTCAAT
CGCATAAATCTGGTAAGGCCGTAAGAGAATTAAGCTTTGCCGCTCTTGGTCGATGACTGTATATT
CACTGACCATTTTGTGGGCCATGGGAATATTAAGGACTTGGCGCGTGAACGCTAACCCGTTTTCC
ACGGGGTGATTATCCCGCGTCCGCCAATTGAACAAAAAGGCTTTATTGAAATGATCCGGTTCGG
CATTCGCAAAATACGCCGTACTATCCGGCGTCATAATCACAAAC
SEQ ID 13 Position 328723..329314 
CGATCTAAAAGCTAAGTTATTTTCCAAGATATCAAACAACTTCTTAACCCAAGAATCTTCCACACAT
AGGACAATAATGAATCCAAATAGATTCAGCTTTTTCCTGCAAACCGGGATCAGTATAAACGTCCA
GTACCGGATAATCACGCATTAAGTTCAGCTGCCAATGGGTATCATCTAAATTAAAAAGATCCGATT
TAGTGTCTCCCCTTACTACATTATGGCAATACACACAACTGTTGTTATACATGCTTCCTTGCTTTTT
GATTTTAAACTCCTCCATTTTGCATATTATAAGAAGATTACTTCTACTTGATATATAGATGCTTTCC
TTGCGAGGGTAAGTCAGACAAGGAAGCATTTCTAACTTGAGATACTTAAGCTTGTCTCAATAGAT
GTAGATAGCGGCTCCCCAATCGGATATTAACAGCTCAACTAGTCAAACCAGATATATAAATGTGA
CACAAGCTGGAATATATATCATTATCTAGATAATTCAAATTGAGCTAATAAAATCAATAAAGAAAAT
TTTAAATAACATTATTTTATAAACCCCTTTAGGATTTTCCCGATTTGATATTCTACGTATGTT
SEQ ID 14 Position 2002858..2005090 
GAGTATCCAAAAATACGACGGGTATTTGAATAGGATACTTATTAAGCGAGAATGGTATTGGAAAT
CTGTGGCAGCCACTCAGCGGAACCATACCTTTATCCCAACCCCACGCAAAAAAAACATCAAGTAA
TCCGTCAGATATGATGACTTAATTGTGGGACAGTTCTAATATGAAGAAAACAGGTTAGATAATTGG
GGTGAAAAGATGGCAACCTATTCGCAGATAGAACTAGACATAATCAAATCATTTAAAGGGCTGAT
GAAAGACCATGAATTCACTGAGATCTCAATTAAAATGATCGCTGAAAAAGCCGATATCACTCGAC
GCGGCTTTTACAATCACTTCTTAGATAAATATGATCTTGTCAGTACCATCTTTGAGCATGATCTTTT
TCCAACAGTCATCAGTTTGACGAATATCAATGACTGGGATCAAGGGTCGCTGTTTATCGTGAATT
ATCTCCAAGACAATCGCGACTACTATAAAAAATTGTTGTCGCTTGAAGGACAAAACTGTTTACAGA
CAGACTTTTATAAATTGACTGAGATGCAGATTGGGATCTTGATCCCAGAAATATTGGTCGGTAGG
AAAATTTCTGACGAAGATCAGGCATTTTTAAGCGATTATTATTTTCACGCTTATATGGGACTGACT
ACCGAATGGGTCAAAGGTAAATATGGTTTTTCAACTCAGGAGTTCGTTAAACGGTGGAAAGCCTT
ACTCAATAATTCAATGCATAATTATCTGGACAACTACGCTCGATGAATTACACAGATTGGATTAAA
TGAGAAAGATGTTACATTTGTGCCAATATGTGAATTGATAAATATTTCACAAGGAACTATTCTTTCC
CTGTAAACGAAAGTTGACTTGAAAGGAGTTAGTTCTGATGAGTAAATATAAAGTTATTATTTGGGG
ATTAGGAAACGTTGGTCGTTCCGCAGTGAGAATGATCGCGGAAAGACAAAATATTTTTGAATTGG
TTGCAGCCGTTGACGTTGATCCAAAGAAGTTAGGTAAGGATGCCGGAGAAGTCTTTGATTTTGAC
AAAGTCGGCGTCAAAGTTTCAGATGATATTGATGCAGCCTTGAAACTTCCAGCTGACATTGTGCT
CGACTTTTGCCCAACGGAAATGGACAAACAAGGAACATTCATGCCTTCTGCTATTCGACTCGCCA
AATCGCTCGATGCCGGTAAAAACGTTATTACCACGATTCCGGTATATCATGTTCAAGACAGTCAG
CCAGAAGTATATGAATATCTAAATGAACATGCTAAAGCACATAATGTTGCTTTTGTACCATTTGGA
CTTTTGCCAGGCGATTATGCCTCATATATCCCACTAGTTTTGGCCGGGGCCATGGGCCACGTGG
 
ATAAAATTGTTGTTCAATCCGGTGAAGATGACTGGCACAACACATCAGGCTGGGTCGATGTCTTC
TCATATGGCGGCGATATCAATAAATATCCAAAACCAGACTCAGACGAAGATCTCTTGGCTAAGTT
CATTTATGCTTATTATTCATCCGGCGTATACGAGATGGCCGATAGGATCGGTCTGAAATATGATA
CCTTCAAACCAGAGCATGAAGTCTTCACTGCACCCAAAGATTTGGAAACGATCAAGGGTACAGTC
AAAAAGGGCAGCATTTATGCCCACAGATTTACCATGGCACTTTACAACGGCAACGAACAAGTAGC
CGCCTTAAGATATGTTCATAAAGTTGATAATAAAGAGACACCAGAATTACCGATCAATAATACGAT
TCATATTGAAGGCTTGCCGTCAGTCGATGCGCAGATCGATGGATTGATCCCAGAAAGAGAAGGC
TACGTTTCATCAGCCGCTCCAGCAGTCAACTTGATCCCTAGCATTCTCGAGACCGACAAGACAG
GTTATGTTGAAGTCTGCGACCTTCCAGTAGTGATTGCCAGGCCATTGGATATTGGCGCAAAAAAA
TTAGTCTAGACTAGGCTTTCGAAGCTGCTTTGACCATTAAGGTTGGAGTAGCTTTTTCATTTGCAA
GTAAATCATTACGGCTTGTGTATACGGTATACAAAATGGAGAAAACGCTGACTAGTTTATAAATCA
TTGAGACTTAACGGCCGGATAAATGCTGATCTGATTATAGAAATAACAACAAAAAGGCCACGCTA
AAAATCATATTAATTATAATCGGGAAATTTATTAATAATATTCAAGAAAAATAAAAACCGTGGGTAC
ATTATTTAAAA
SEQ ID 15 Position 2262750..2268615 
TTTGAAACTAAGACGAAAGCTGCCATGTCAAACAAAGCCGCCATAAATGCCACTGTCACA
GATCCATCAGCCGCAATGCCAGCATCTTGCTGAAGTTCTTTAACAGCATTAAGGGTGTTA
TTCGTGAACTCATTATTAAAGTCTACTGGACTAATCCCTTTGCACCAGAAAGCCCCTTGA
ATGAGTTGAGCAATGTTCCCCTTATATCCTGGCTTCAGACTACCTACAACAGGTGCTAAG
GCGTTTTTGGTCGTCTCGCCAAAGCCTTCACCAATAGCACTAATACCGATTTCGTGCTGT
AATCCCATTCTTAGGCTATAAATTGTTGGCCATCCCGTTTGCCCGTTTTCTGGAGCTGCG
ACAAAGCCAGGAACGCTACCATACGTTTTGTTGAGCCATTTTTGAACGGCTCGTACTGCT
TCATCTGCCATTTTAAAGTCTCCTTTTTTGTTTTAGACAGCACGTCTGCCGTCACAAAAA
GCAAACATATGTTCGGATTCATTTCATCTCTTCAAAGCTTCGAAAGGCAACCCTGATCCA
CAAATAATCCTTTTATTTTGAACTTAGCAAAAAAATGAGGCCCTCACATAGTGTTGAAGT
TGCCTCATTCTTAATGTCTATATTTAAAGTATTGCCACAACGATGGATCATCGAACGCTC
ATGGACTTGGTTAGACATGTATCGACGACTATGAAAATGTGGGCGCAAGCTCAATTTCAC
CTTCCAAATGTTTGTGCTATCTCATTTAGCGCTGGTTTTTTTAGGATAGACTAGACAAGG
ACTAATAATTTCTCAAGAATCCCGCAACTCCACTATTCATTCGTCGAAATCCCCACTGAT
ACTCTTGTCCTTGCACGTTCGACCAAGCAAGAATGTTTATTCCGATAACCGAATTGTTGC
CATCAAGTAATGGACCTCCCGACATGCCATGATACGAATTAATTTGTTGTGAAATATATA
TTCCTAGTGGGTCCTCTGAAAAGGGTGTGACAGTCCCACTTGATTGAACCATGACTCCTT
GAAGTTCGTACCCTGATTGCGGATCGCCAGGGAATCCAATGGATCTTGCTGCCATCGTAT
CAGCAGGGTTCGTATTTAAATTAAGACCCGCAGGCATACTACCAGACTTCATAGAGACAA
TTGCAGCCCCGTAATCATTCGAAGTAGCTGTTGAATTATTAATCCATGCCTGTGGCACTA
TCAATCTATTCAATACTCCGTAACCGACCCCTTGATGATTTGCTTGACTATCACCAAAGT
TAATAATTCCTCCAGAAATATAATGACCATCATATAACATGTGTGCTGCTGTCCCTATAC
GGTCTACTCCAATGCTAAATCCAGTACCGCCAGAAGTTCCACTGCTCAGCTCTGTGCCAT
TTGAGTTAGAGATGATTTTTTTATACGAACTGTCAAGGTATGGTGAGTTAATGACCATGA
CACCATTTGCCATCGAAAACCACGTGCTCAAAACCCCAACGGAACTGTACGGCGCCGAAT
TGGGGTTTGACACAGGTGATACCGTCCTCACAGATAGATGATTAGACGATTTATTCAGTT
TCGCAAGATATTCGGACGTAATTCCTTGAACTTTCTTTCCTTCTTTTAAATCCTGATATT
GATCAGGTGTATAAGCTTTGACACTCCCTTTAAAATCGGGATAAGAATACTGATATTGTC
GAATGATTTCATTCAAAAACTGTTGTGTTGTCGTCTGGTTAGTCAAAACAATATCATTCT
TTGGTAAAACATGGCTATTCGCTGCCAAAGGATTTGCCATACTATCAGCACTTACACTTA
CCGTTTGAACTTGAATTATCGCTAAGGCTGCAGCTATCATAGTGATATATGCCCATACTT
TTCGCAATTTAATTCCCCCTTTTTCTTAAAATGAAACCGCATTCACGGAGGCTTGTCAAT
GCTTTTAAAAAACAAACGTTACTTTTGGCTCATCTTGGCTGTCAGCATAATTGGAGTAAT
TGTTCTTGCGGTGTTATGGCGCATGAACCCTGAAGGAACGGCCTCAAATAAGTTTGAACG
TCCCACCATTACTATTAAAAAAGTCAAACTTATTAAGCACAGTAACAGTATTGCTGTCAC
ATTTGCTACCTCTCCAAAAAGCAAATATACGATAAGTGATCTTAAAGAGAATCAACTTTC
TTCTGGCATTTCGAATAAAAGAGAAAATACCGTTTCGGAATTAAAGCCCTCCTCCTCTAA
GCTTGCAATACGGGTTAAGCATAACAATAATATACAAACCAAAGTGGTTTCTGTTCCCAT
TGGTTATCATATTATGAAAAGTGCCATTTCAAGAAAGCCAATTCCTATGGGAGAAGAGTT
TAAGTACAATGGAAAGTCGCATGTTTTATTTAGCATGACCATTACCCCTAAAAAACAAAA
CAAGAACAGTATAAAAAACACCACTGCTTTTAACATAACGGTTAAAAATGATCACTACCT
TGTCCCTGTCGTATTAGATACCAAATACCTTACTGTTTCTGATTCAGAAGGTAACTCATT
AAAAGTAAAGCCATTCTCAAAAATTTCTATTCCAGCAAAAAAGAAAAAGACCATTGCAAT
AACTATTGAGGGCGTTCCCGCAAGCTCTGCCAATGGTCTAGTTATAACGTATAATACTGT
 
CGATTTAGACTTACCAATCTCCTTTATAAATTCCTGAAATTACACTAACTGTCCCCCACC
TTGACAGTCAGTACACTCAAACTGTCTCTTATGCTTACAAACACGTAATTTAGGCGGTTT
TTAAGCAAAAGTCGTTAGTTTTCATAAATGTTATCTTATACTCTAATGAGATCTAGCTTG
TGATAATAAGGCTGTTTTTCTTTGACAGCCTTATTAAGCACACTAATCAATGTCAATTCG
AAGTTTTTGGTTTCCTACTTGGCCAACTTTGTTATCAGAAATTCCAAAACTCATTGCCTC
CCGCCACCATATATTTATCGAGCCATTTTGAAAATGAAAAATCGAAATATCGGTCTGCTT
CTATTCCGGGATGAGTTAGATATGATTTTCCTAACCGATACTTCTCTATATCAATATACA
TATCTCCGACATCCCTTATATGGAGAATGGGTACTTTATTTACAAAACTTTTAGGCAGTG
CCTCTTTTTCTTTAATCATTTTTCTAATTGAATAGACTTCACACGTATATCCCATAGGAA
TCCCTTCGATTGTTGTGTCAAATAAAAATAGTCCATTAGTAATCGAGAGAAACTCAATGT
AATCCTGTGGAAGGTTCCACCTTTTTATTTTTTCTATATCATCAGCGTGTGCAGGAGGTT
CTATCTTAAAAGAAACATTTTGCACATCTCCATCTAATTGGAATGTTGAGAGCGCTTTTT
CCCCATTTTTCGTCACCTTTATTAAAGAATTAATTCTTCGCCGAATTAGAGATTCCAAAT
GAGTTCCTCCTCAATAGTTGTTAAACCACGCGGTGATCAACCGATGATTTGGTGTTAACA
CCGGCATCAAATTATTAAAGTCATTTGTTCCGCCATAAACTCTCGGGCGAATATGATGCA
CTTCTCGAGAACTCCAAAAATCTGCAGATTGATTGCCATATGTTTCACTGAACGTTTTAA
TATAAATATATCTATCTTTTGATGACCAACTAGGAGATTTTGATAACTTAGTCCAGGTAG
TATTAATAGGTGTTTCTGCATCTTTTTTAGACACGGGATCAACATATTCAGGGAAATTCT
GTCCTATTTTATTTTGTAAATAGATGGCCGTTGGTGGGATTGGTTTTACATTAGCCGCTC
CATTAACTCCTATAAATCCAACAGCGCCAGCAACGTTAAAAAATGTGGTTTTGGCTGGCA
ATTCTGTAAAACCAAGTAATCCTGTTTGCAAACCGCCTATTTTGCTAATGGGTGCCAGCG
TATTGGCATTCACCGGACCTGCAAGGGTAGGCTCAGTATTAAATTCAATTTGCACATCAA
CTGCGGCTGGCGGGACACCCTCAATAGAATCAATCCAGAAATTGACTCTGAATTTTCCGG
ATATGTATTCTTCCGATAAGTGCCAGGTAATATTTGTAACAGGAACTTCTGCACGAGAAC
TTATGCCATTGCCATGACTATTATTCAGTACCACTTGTTTTCCAGAAGCGATAATTGGTT
GTCCATTTTCTGGATTACTTCTTGATAACTTGTCAGCATTGGTCAGTTGAGTATTGTTAT
CGCTAATACGGCTTTCAGTTGAAACAATTTGATTTAAAGATTGTGTTGTGTCTGCGGATA
CAATTGTTGAATACCCAAGCAAATTGACTAGAAAGAGCCCGAATATTAGCCCAATAACTT
TCCACTTTTTCACGCCTATTATCTTCTTTCCAAAGTTCTTCAGTGCCTGGCAATAACTGT
ATACATTGAGCAGTATAGTCGCTATTTTATAGCTGAACAACTCATAAAGCTCAATTATTA
TTAGCCTATAAAACCACTGCCTAAGTGAATTGATCTAGAACGAAGCACGCCGAAGAAGTC
GCTAAATGTGCTAAGAAAAATGTGCTTGAATAGCTCAAAAGTAATTAGCGTCTCCATTGA
AAATCCGTTATTTTTAAGTGATCTAGTGTTAACTATGAATCCCAAATAAAAAGCAAAATC
CGTAAATGCCAAATTTTCCTTTTTGACGTTTTTCTACTGTCGCGAGATTTGCAAGTGTAC
GTACACTTACGATGAATTGACAGAATCTCAGCTGCGCTGATCGTCAATTTTGTTTGGGGG
CACGCCCCCAATCCCCCTGTTATTTTGAAGGGAGGTGAGTCCCCCTTCAAAATCAAAATT
TAAACAGCATCTGCCGCCATCTTTTCGCTGACCTTCTCACGATGTTACACGTGGTGTTGA
CACCCACTTGCATTTAGAGTTTCATTCAAGTTGAACATTGTGTAATATATGAGTTGCATT
TGATAAACATATCAGTTGCTATTTGTGCAACTTTAAAGCTTCGGCTAATTCAACGTTCTG
TTAATTTACAAGCATCTCGACAGTTTCTGTTAAAGCAACATCTACGCTTCAATTCGAGCA
ACTCACTATACGTATGCCGAGTTGCAGACAAGCTACTATATAGCTGTACGCGCTGAAACA
CCAAAAATCGTTCGTTTATGCCCAATAAGCGAATAATCTTGCTCAGGTGTAGTAAAAAAC
TGTTTACGTGTAGTGAATGGCGCTAGCCCTTGTCGTAACTGGCATCATCCACGTGTAGTA
AAACGCGTTTTACTACACGTTCGTAATTTTTTCACGTGGAGTAAATGGCGTTTTACTACA
CCTTTTGACCCCAACGTGCTATCACGACAAACCAAACCGCACTGCGGTTTACCCCAATTT
TGGGGTCAGTTTTGCCTTATGCTCTTTCATGATTTTAGGCGCGTTCCAAGCAGTCTCAAA
AAGTGGTCGATCCAGGCGAGCCGATTTTTGAGAAGGATTGGATAGCAACTCAATTTATTT
TGATCTTTTGCTTGGAGAAAAACGTTCACGTTTTGACCAGGGCCGTCGCAACTGTTGACC
AAAACTCGTCCGGTAACGTGACGCTATTTAAACGCCGCGTTGGTTTGCTAGACGACCATT
CATCATCACCATTCAGGAGGTTTTTGAAATGACAAAGCAAGACGAAACACACCGGGTCAT
GTTCACTTTGACCGATCAGGCGATTGCAAAATTGAATCAGCTGGTCGCAAAAAAGCAACA
GGAAGTGAATCAAAATCCGGAACTGGCTAAGTACCATGTCAGCGTGACCAAATCAAATAT
CATTGAGGACTGGTTATCAAAGCAGTGAGTTTAAAAAGCGCTAAAGGGCCTGTACTAGCG
TTTCTTACTCTGGTGGGTATAATTAATGCTCTCTACATCAAAAACG
SEQ ID 16 Position 2776965..2787971 
GCCACGAACCTGTAGCCGTTTGGATGAAGCCATATAATACTGGACCAACCGCCGCAAATA
AGTAGCCGACACTTTGAGCAAAATCAGGAATACTAATCTACTTTGCCCTTAAAAAATCTT
GAGATGATCCATATCTTGTTTTGCCTTCATTACTGTAGTTGGTCATAAGAAGTGCCCTAC
ATTCATTAGATTACTTGTCTAATAATTGTAGGGCACTTGGGTTGAGAAAAATGATGTTAA
 
CTAAGAATGCAAACGAACTAAAATCTTTGCTTGCTTTTTATCCTTTTCTAAGGATTCAAT
TCCTTCTGAAACTAATTCATTTAATTCAATCTTTTTTGTAATGACCTGTTTGAATAGTGA
ACGATGGGTATCTATAATCTTAATTACTCGATCGAAGATATTGGCATATCCATAAGATGT
TAATAAACTACCACCTTTTTTAAGAAGAGCTCTAACATCTACAACTGGTGGATGTTGAAA
TAATGCAATCACGGTAACCTTGCCACCATTTTTAAGAGCCTGAATGGCACCAGTAAGTGT
GGGTTGTACACCGGCGCAATCAAACGCAATATCCACTCCCTGATTTTCCGTGATAGTGCT
GATAGCGTGAGCTAATGACTTTTGACTATCAGCACGTATTGGGTATTGAATTCCTAATTC
ATTTGCTAAATTCAAACGTTCCTCTGACATGTCATTTATTATGACGTGATGTGCACCAGA
AATTTGTGCTATTAAGGCCGTGAACAATCCAATTGGACCAGCACCTTGAATTAAAACATC
ATCTCCAGGAGACACTCGGCTTGCCATAACTGCCTGTGCAGCAACTGAAACTGGTTCAAC
TAAGGCCCCTAAATCAAGCGGAAAGCTAGCTGGTAAGAGATGTGCAAAGGTACTTTTTAC
ATTGCACTTTTCAGCTAAGCCACCGTTAGCCGAAAATCCTAAGAATCCTGCTGATTGATC
ACTACCTATAGCATGTTCACACCAATTATAATGACCAGAAAGACATTCCGGACATTTTCC
ACAAGCAATCATTGGTTCGACTGCAACTTTATCTCCAATTCTTAATTTAGATACTTGTTT
TCCAATTTTAGAAATCGTCCCAGAAAATTCATGACCAGGAATTAGCGGGGCTTGCATATG
GGTTAGCGGATGAGGTATTGTCGCCAAATCCATACCCTCTAAATATTCATGAATGTCACT
ACCGCAAATACCATTAAATGCAACCTCAATTTGAACTTCATCTGGTGCGGGATCAGGAAT
ATTTCTTTTTTCAAAGCGGATATCCTTAGGACCGTAAATAACAGCTGCCTTCACCATAGT
CATAGTGCTTCGCTTCCTTCATGTTCAATATAGCACAATCGTATATAAAATAGTGAATAG
ATTTCAGTAATGAAGTTACCATCTTGACTTAACAAAAACTTGCTAACTGATTATATGAGA
AACTTTTACTTGAAACATTTTTGGTGATTACCATTAATTCCCTCGGACATATTTTGAAAA
ACCCTATTTGATGCTGATTGCAAATTATTTTATGCGTATTTATTAAGGGTTTCTATGTTG
AAGTATATAGCAAACTTGTTCAAGTAACTGACTTTCACGTGGGCTTTAGCCAAGAGATGC
TGAGCAGCGAACCCAAGGGGTGTTACTCGCCCACGCAAAAAAGAAATCCAATTGCATTCC
AGTATGAGCGAGAAGCAAGCCATTAAGACGCTGATTCATGAACTCGCGCACAGTGAATTA
CATTGTGATCCGAAGTTAAAATTGGATCGTTCAACCATGGAATTGGAAGCTGAAAGTACC
GCGTTTATCGTTTGTCAACATTTGGGAATTGACACGAGTGATTATACGTTTCCTTACCTT
GCTGTTTGGTCGAAAGATAAGGATCTTTCCCAGCTCTCCAAAAGCTTAACGCGTATCCAA
TCCACCGTCGAAAAATTCAATAAAACCGTCGATCAAAACCTTGAAAAGATTCGTGAGAAA
CCGTTGACGCTTGATCAAAAAATAGAACGCGCTAAAACCATTGCGACAACGGAAAACATC
GCAAAAAAAGAGCAAGGGCTGGTGCAAGCAACGCAGGAGAAAACACGCTAACCCATTTGT
TGAATACTCTCACTCAAGAGGACACTCCAGCCCTTGATCACCCAAGAAAGGAATTACCAA
CATGAAAACCATTGACGAAATGAACGAATTCGATCGTGACATTATCTTACTTCACCGCAA
GTCTGTGAGCGAAGATACACCGCAGGCAATTCTTGTGAAAGTGAAACAGATTCGTAACGC
AATTGCCGACGAAAAGGCGGGTAAAGAAGATCCAATTGAGAAAGAATTTACACTCGAATG
TTACGACGAAGCAATCAGAAAACTAAGGGACCTTTCGGTCGCTGATTATCAGTTGTGGTT
GCGTCAAAACAAAGACCTGGAAGGGTTTGAATTTTGATTTTGAAGGGTGTCGTAGACCCC
TTCAAAATAACGGGGGATTGGGGGCGTGCCCCCAAAACAAAATTGACTATCAGCGGAGCT
GATATTGGATCAATTTATCGCAAGTGGACGTCCACTTGCAAATCTCGCGACAGTAGAAAA
AGCCCCAGAAAAGCAAATCTGAAAAAATGTAACAGGCACTTGATATCAAGTGCCTTATTG
TTTCTAGGATCGCTAAAAATAACAGGAGGTGGTTACATGAAGCAATCTGATGAACACCGC
ACGCGTTCAGTGAGAAGCACTGTGCGTATGACCCCAGAGGAGCGTGCTTGGGTTGATATG
AGAAGAGCCTCTGTCGGCAATCCAAAGTTCAATGCATTTGCCTGTCGCGCACTCACGACG
AGCAAGATCGTTCATGTACATTTTACTGATACTAAAAAGTTACTTAGACAGCTGTCAAGA
ATTGGGAATAAGGCTCCTATGCTGTAATTACGGACAAAAATAGTTTGTGCGATAATTACA
GCATAAGGGCCTCTAGGTCGGAGCCCAGGAGGCGGAGACCGCCGCACAGCCCAACCCCAC
GCCGAACCGGAGGCCAGCCCGCCCGCACCGCGGCCGCAATCATCCACCCAACGCCCCCCA
AGTTTTTGATAGCGGTAACAACGCCTGTGCGCTTGTCGTGGCCGGCCTTTTTTCATAAGG
TTGGAGGAGAAAGGAAGGGTGGTTATGGGCGCTTGGTATGAACACGCAATTATTTACCAA
ATCTATCCAAAATCGTTTCAAGACAGCAACGGCGACGGCATCGGGGACCTGAACGGGATC
CGGCAACGGATCCCGTACCTGCAAGCCCTCGGCATCAACACGGTGTGGCTGAACCCGATC
TTCGTCTCCCCACAGGTGGATAACGGCTACGATGTTGCCAATTACTTCGCCGTGGACGAA
ACCATGGGTACGATGGCCGACCTGGAGGCGCTGATCGCGGCTCTGCACGCGGCCGGCATC
CGTCTGATCATGGACTTTGTGCTAAACCACACCTCGGATCAGCACCCGTGGTTCCAGGAC
GCCATTCACGCCAAAAATAGTCTGTACCGCGACTACTACATTTTCTCTGGCCACGACGGG
CAGCTGCCAAACAACTGGGGCAGCTTCTTCGGCGGATCGGTTTGGGCGCCGGATCCGGCG
GGAACCGGGCAGTCGTATTTTCATCTGTTCGACCGGCGGATGCCGGATCTGAACTGGGCC
AATCCCGAGGTGCGGCGGGCGATGGGAGACGTCGCCACGTTCTGGCTCGGCAAGGGCATC
GACGGACTGCGGCTGGATGCCTTCATCCACATTGCCAAGGCCGATCTGGGGCAGGATTAC
CCCCTGGCTCCGGGGCAGCAGACGCCGGTGGTGGCGGAGCCGTTTTTCTCCAACCTCCCG
 
AAGGTGCAGGAATGGCTGCGGCCGTTCTGCGACCGGATCAAAACCGACTACCCCGACGCG
TTTCTGCTCGGCGAGGCGGCATCGGCCAACGTTAACCTGGCGGCGGATTACACCGCGCCT
AGCCAGCACCTGATGGACAGCGTGATCACGTTCCGCTACTTCACCGAGGACGAAAGCGGC
CTGGATCCGCGGCTGCCGGCGCAGTACCAGCCGCGGACGCTGGATTTCCCGGCGTTCAAG
CAAACCCAGGCGGTGTGGCAGCAGACCCTCGCCGGGGTGTCGATGCCGACGCTGTACTGG
GGCAACCACGACATGGCCCGGCTGGCGACGCGGGTGGCCAAAACCACCACCCAGGCGCGC
AGTCTGGCCATGCTGATGTACCTGCAGCGCGGCCTGCCGGTGATCTACTATGGCGAGGAG
CTCGGGCTACACAACCTGCAGTTCGATCACGTTGATCAGTTTGCGGACGTTTCGGTGGCG
CCGTTCGTGGCCGCGGTCGAGGCCACCGGGCAGTCGCGGAGCGCGGCGCTGGCCATGGTG
TCGGCGACGCACAAACTGCCGGCACGGGGGCCGATGCCTTGGACGACCGGGTTGCACCAG
GGCTTTTCCAATCACCTGCCGTGGCTGGTTGGGCGCAGCGAGGACGTGACCAGCGTGGCC
GCGCAGCAGGCCGATGAGGCCAGCATGCTGCACTTCTACCAAGCGCTGATTGCCCTGAAG
AAGCAGCCGCTGTTTCAGGCCGGGCATTACCGGCTGCTGACGACGGCGCCGAACCTGTAC
GTCTACGAACGCACGCTGGCCAGCCGGCGGGCCCTGGTGGCGGTGGCCTTGGATGAGCAA
GGCGCCACCTTCACCGTGCCTGAAGGCCTGACGACCGTGGCGCTGGCCGCCGGCGATTAC
CAACTCGAAGGTCAAACGCTCACGCTTGGCGCGAACGCCGGCGTGGTGTTAAACGAAAGG
GGAACTCGATAACCATGCAACTTGCAGCATTACGGCACCGCCCAGAAAGCGAAGATTGTT
TTTTGTACACTCCAGATGAGCTGCGGCTGCGGCTCCACACAGCCAAGGCCGACGTGCAGG
CGGTCATCGTACTGTACGGGGATCCGTATGTCACCGCGCCGAACCCGACCACCGGAGAAC
CGGAATTCGCCTACCAAGAGGCGGCGATGATCAAAACCGGCACCGGCCAAACCAGCGACT
ACTGGACCATCAGCCTGACCGCGCCTTATCACCGCCTGCAGTACCAGTTCCTGGTGACCG
GTCAGGACGGCAACACCGTCCTGCTCGGCGACCGCGGCTTGCGGGCCGACAGCGCCGCCA
ACCGCCGGGCCGATCTGTTCCGGGTGCCGTACTTCCACGCCATCGACACGGTACAGACGC
CGGCCTGGGTCAAGGAAACCGTGTGGTACCAGATATTCCCGGAACGCTTCGCCAACGGGG
ACAAGACGAACGACCCCAAGGGCACCAAGCCTTGGCGTCCGGCGGATCACCCGGGCCGTG
AGGATTACTACGGTGGCGACTTGCAAGGGGTGCTGGACCACCTGGACGACCTGCAGGCGC
TCGGCGTGAACGGGCTGTACTTCTGCCCGGTGTTCACGGCGATGTCGAATCACAAGTACG
ACACCATCGACTACTTCAACATCGACCCTGCGTTTGGCGACAAGGCCTTGTTCGCCGATC
TGGTCAACCAAGCGCACCGCCGCGGCATGCGGGTGATGCTGGACGCTGTGTTCAACCACA
TGGGCAGCCGCAGCATGCAGTGGCAAGACGTGCTGAAGTTCGGTCCGCAGTCGCGCTTCG
CCTCCTGGTTCCACATCAACCGTTTTCCGGCGGCGCCCTTCGCCGCGCCGGAACAGGGCG
GCGTGCCGCAGTACGACACCTTCGCCTTCGAACCGCACATGCCGAAGCTCGACACCAGCA
ACCCGGCGGTGCAGGACTACCTGCTTGAGGTGGCGACGTACTGGATCAAACAGTTCGACA
TCGACGCCTGGCGGCTGGATGTGGCCAACGAGGTGGACCATCACTTCTGGAAACGGTTCA
ATCAGGCAACCAAAGCGCTCAAGCCCGATTTCTTCGTGCTGGGCGAGGTCTGGCACTCCA
GCCAGCCGTGGCTTAACGGGGATGAGTTCGATGGGGTCATGAACTACGCGTTCACCGAGC
AGATCGAGGCCCACTTCCTGACCGGCAAGCTGAGTGCTCCTGAGCTGACGGCGGCGCTGA
CGGATCAGCTGATGCTGTACCGCGACCAAACCGACCAGGCGATGCTGAACATGCTGGACT
CGCATGACACCGCGCGGCTGCTAACGGTGGCCGGCGGCGACGAGGACCTGGCCCTGCAGG
CGCTGGCCTTCACCTTCCTGCAAACCGGGATGCCGTGCCTGTACTATGGCACGGAAATGG
GCATGGCCGGAGAAAACGATCCCGACTGCCGGCGGCCAATGGACTGGGCCCAGCTGAAGG
GCCCGATTTGGCAGCGTGTGCAGGCCCTTGTGACCTTCCGCCGCGCCCAGTCGGCAACGC
TAGGCACCGGCACCACGGCGCTGAGCGTGACCGCAGCCGGGCTGCTTAAGGTAACCCGCA
CAGGTGAGCACACCGTGACGGCGTATTTTAACACCACCAAGCAGATGGCGACACTGACCG
TCAGTCCATTACTGGCGCAGGGTTACGCCGGCCAGCGGCTGGCGCCAACCGGGTTTGCTG
TTATGGTTCAGTAAGATTATGTTAGCGGTAACAGGCAATTTGACCTTTTAAAAGCGTTTT
CATATTATCATAATCAAAAGTGTAGAAAAGTTCAGGTGGCGCAATTCACCTCCCGAAAGT
GAAGGATGCAAGATGAAACGGATATTTGAAATCGACCCGTGGCTGGTGCAAAGCCACCAA
TTGAACCCCAACGAGAAACGCCTGCAGGAAAGCATGACCGCCATCGGCAACGGCTACATG
GGTCTGCGCGGTAACTTCGAAGAAGGTTACAGCGGTGATCACCTGCAAGGCACGTACCTC
GGCGGCGTCTGGTTCCCAGATAAAACCGTCGTCGGTTGGTGGAAAAACGGCTACCCGGAT
TACTTCGGCAAGGCGATCAACGCGCCGAGCTTCATCGGCATGGCGCTCACCGTGAACGGC
GAGCGCGTCGATCTGGCCACCAGCGTCTACCGCGATTTCACCCTCACGCTTGACCTGCAC
CAGGGCCTGCTGACCCGGAGCTTCGTGTTCGAGGGCAAAAAGGCCACGGTGCGCTTCACC
TTCAAGCGTTTCCTCAGCAACGTAATCAAGGAGGCGGCGCTGGTGCAGCTCACCGCCGAA
AGCCTTGTCGGACCGGCCGAGCTGACGGTGGCCGCACAGCTCGACGGCAACGTCACGAAC
GAGGACAGCAACTACGACGAGCGCTTCTGGGCACCGCAGGGGGAAAACGCCGCGGCAGGC
ACCATCCAGCTGCAGACCAAGCCCAACCCGTTCGGGGTCCCGCAGTTCACGGTGCTGCTC
AAGCAAAGCCTGCGCCAAGGGGCAACCCTTTTACCCGGCACCGTGACCACCAGCACCGGC
CAGCTGACCAGCACGGTCACGCTGCCGCTGGCGCCAAACGTGCCGGTCCAGCTGGAAAAG
 
GACGTCATCGTGGTCACGAGCCGCGACGTCGCCCCTGAGGCCCAGGCCGAAGCGGCCGCG
GAGCTGATGACACAGCTGCAGGGCCAAAGCTTTGCGGCCCAGCTGGCGGCACACACCGCC
CTGTGGGCCAAGCGCTGGGCCCAAAGCGACGTGGTGATTGAAGGCGACGACGCGGCCCAG
CAGGGGATCCGCTTCAACCTCGCCCAGCTGTTCATGACCTATTACGGCGACGATAAGCGG
CTCAACGTGGGGCCGAAGGGTTTCACCGGCGAGAAGTACGGCGGGGCGACCTACTGGGAC
ACCGAGGCGTACGTGGTGCCGATGTACGTCGCCGCCACCCCTCCGGCCGTGACCCGGGCA
CTGCTGCAGTACCGGCACGACCAGCTGCCCGGCGCCTACCACAACGCCCAGCAGCAGGGG
CTCAAAGGGGCCTTGTTCCCGATGGTGACCTTCAACGGCATCGAGTGCCACAATGAATGG
GAAATCACCTTCGAGGAGCTGCACCGTAACGCAGCGGTCGCCTTCGCGATTTACCAGTAC
ACGGCCTACACCGGCGATGAAAGCTACGTCAACCACGACGGCATGGAGGTGCTGGTGGGC
ATCAGCCGCTTCTGGGCGGACCGGGTCCACTTCTCCAAGCGCGCCGGCAAGTACATGATC
CACGGCGTCACCGGGCCGAACGAGTACGAAAACAACGTCAACAACAACTGGTACACCAAC
ACGATGGCCGCCTGGTGCCTGGAGTACACGCTGGCCCGGCTGCCGAAGGCCGATGCCGCC
ATTCAGGCCAAGCTGGCCGTGAGCGCCGCCGAGCAGCGCCAGTGGCAGGACATTATCGAC
CACATGTACTATCCGGAGGACAAGAAGCTGGGCATCTTCGTCCAGCACGACACCTTCCTG
GATAAGGACCTGCGGCCGGCAAGCTCGATTCCGGCCGACCAGCGGCCAATCAACCAGCAC
TGGTCCTGGGACCGAATCCTGCGGTCGCCGTTCATCAAGCAGGCGGATGTGCTCCAGGGC
CTGTACTTCCTGAACAATCGCTTCACCCGCGAGCAGAAGGAACGCAATTTTGACTTCTAC
GAGCCGCTGACGGTGCACGAAAGCTCGCTGAGTGCCTCGATTCACGCGGTGCTGGCCGCC
GAGCTCGGTAAGCAGGATAAGGCCGTTGAACTCTATCAGCGTACGGCTCGTCTGGACCTG
GACAACTACAACAACGATACGGCAGACGGTCTGCACATCACCTCGATGACCGGCGGCTGG
CTGGCTATCGTGCAGGGCTTCGCCGGCATGCGCTACGACCACGATCAGCTGCGGTTCGAT
CCGTTCCTGCCGAAGCAGTGGCAGGGTTACCAGTTCCGCATCAACTACCGCGGCCGGGTG
ATCCAGGTCGCGGTGGGGAAAACCGTTGCAGTGACCCTGCTGGCCGGCCCGCCGCTGACC
GTCATGGTTGCCGGCCAGCCGCAGCATTTGGAGGTGAGCGCGCATGCTTAAAGGATTGCT
GTTCGACCTCGACGGCGTCTTGACCGACTCGGCCAAGTTCCACCTGCAGGCCTGGAGCCA
GCTGGCCACCCAGCTGGGCATCACCCTGACGCCGGCCGAGCGCGAAGGCCTGCGCGGCCG
CTCGCGGCTGGACTCGCTGAACCTGATTTTGGCGGCAGGCGCCCAGGAAGACCGGTTCAG
TGCCGCAGAGAAAACGGCGCTAACCGACCAGAAGAACGCGGTGTACCTGAAGCTGATTCA
GACGATGACGCCGGTGGACATCCTGCCGGGCATTCCGCAACTGCTGAAGGACGCGCAGGC
GGCCGGCCTGAAAATGGCAATCGCCTCGGCGTCGCGGAACGCCCCGACAATTCTTGACCA
CCTGGGCCTGGCCGCCAGTTTCGACGCCATCGTCGATCCGGCGACCCTGCACCGCGGCAA
GCCCGACCCGGAGATCTACCAGCAGGCGCAAGCGCTGCTGGGGCTCCAGGCCGCCGAGGT
GATCGGCTTCGAGGATGCCTCGGCCGGGGTCGCCGCCATCAAAGCGGCCGGTCAGTTCGC
GGTTGGCATCGGGGATGCCCGGCTTCTGGCCGCAGCGGATTACCTAGTGAAAGACACGGC
GGCCCTGCAGCTGAGCCAGTTGCAAGCGGCGTTCGCCAAAGAAAGTGGGGAGACTAATGG
TTGAAATCGACTTGGACCACCTCTACAAGAAGTACGACGACGGCGAGGATTACTCGGTGG
TGGACTTCGACCTTCACATCAAGGATAAGGAGTTCATCGTGTTCGTCGGCCCCTCGGGCT
GCGGGAAGTCCACCACGCTGCGTATGATTGCGGGGCTGGAGGACATTACCAAAGGCGAGC
TGAAAATCGACGATAAGGTGATGAACGACGTGGCCCCCAAGGACCGCAACATCGCCATGG
TGTTTCAGAACTACGCCTTGTACCCGCACATGTCAGTGTACGACAACATGGCGTTCGGCC
TAAAGCTACGGCACTACAAGAAGGAGGACATCGACAAACGCGTGCAAAACGCGGCGGAGA
TCCTCGGCCTGAAGCCGTTTCTCGACCGGAAGCCGGCCGCCTTGTCCGGGGGCCAGCGGC
AGCGGGTGGCCTTGGGCCGGGCCATCGTCCGCGACGCCCCAATTTTCCTGATGGATGAGC
CGTTGTCGAACCTGGACGCGAAGCTGCGGGTGTCCATGCGGGCGGAAATCGCCAAGCTCC
ACCAGCGCCTGAACACCACCACGATTTACGTGACCCACGACCAAACCGAGGCCATGACTA
TGGCCGACCGGGTTGTCGTCATGTCCGTTGGCCACGTGCAGCAGATTGGCACCCCGGCCG
AGATTTACCAGAACCCGCGGAACCAGTTCGTGGCCGGGTTCATCGGGTCGCCGGCGATGA
ACTTCTTCAACATGACCTACCAGGACGGCTTCGTCAGCGACGGCCAAAGCATTCGCCTCA
AAGTGCCGGAAGGCCGGGCGAAGATTCTGGACGACCAAGGGTACAACGGCAAGGAAGTCG
TGTTCGGCATCCGGCCGGAGGACATCCATTCGGAGGAGGCCTTCCTGGAGACCTGGCCGG
ACGCGGTTATCAGCTCAACCGTGTCGGTGTCAGAGCTCCTGGGCGCCACCGAGCAGCTTT
ACCTGAAGGCGGATGACACCGAGTACGTTGCCAACGTCAACGCGCGCGACTTCCACAATC
CCGGGGATCATGTGAAAATGGGCTTCGACGTCAACAAGGCGCACTTCTTCAACAAGGACA
CGACCATGGCCATCGTGGCTAAGCCGATTCCGCTGGAAGGCTGAGGAGGTGAGTGCATGA
CCCCATGGTGGCAGCAAGCCGTCATTTACCAGATCTACCCGAAGAGTTTTCAGGACAGCA
ACGGGGATGGCATCGGCGATTTGCCGGGGATTACCAGTCGCCTTGATTACCTTAAGCGGC
TGGGCGTCGATGCCCTTTGGCTGAGCCCAGTGTATGTGTCGCCCGGCGAGGACAACGGCT
ACGACATCGCGGACTACGAGGCCATCGATCCCCAGTTCGGGACGATGGCCGACATGGACG
CCTTGATCGCCGCCGCCAAGCAGCGCG
SEQ ID 17 Position 2793833..2794809 
CCCGCGATTTTGGCGTGATTGGCTTCGACGGGGTATTCCTGGACCAGGTGTCCAACCCCA
AGCTGACCACGGTGAAGCAGCCCGTGCAGCGCCTCGGCGAACTGCTGGCCCGCATGCTCC
TGCAGAAGGTGGCACAGTCCGGCGCCCAACAGGGGGAGCTGCTGGTCGATCCTGAGCTGA
TTGCTCGGGACACGACGCGAAAGTAGATCGGATTTCAACTGTCCTTACCGCTATGGTAGG
GCCAGTTTTTAGGCTCTATGTCAAATCTAATTCATAGCTAATAGTTGATTTGGCAACGCC
TAAGGCGTCAGCCATATCTTGGTAAGTATGATGGCCTTCACTGACCAGTTGAGCTAGCGC
ACCACGTTGAAAACGTGATAAAGTAGAAGTACCCAAAGTAATCACTCCTTATAGCTGGTT
GGAATTAACTACTCCATTGTAAGAGATTGCTTTGGGCCTTTTTTTATTTTTGTTCGGATT
AATTATAGAATTTGTCTAATTAGTTGAAAATTCTTAGGGTTGCCCATATATCTTTTAGTC
TGGTCATTAGTTTTTATGTTTGATCTGCTTTTTTCTGATCGCAAACACCCACAACTGCGA
GTGAGTCCTTTTTGAAGTCGTTGACTGTCAACAATACATTTATTTCCACATTGACATTGA
CAGAGCCAAAGCGCGTTGCCATTAGAACTGCTTCCAAAAAAGCTAATAACTGTGAGTCGC
CCAAACGTTTGATTAGCTAAATCGATACGCTTTTGCATACTAATCCTCCCGCTTGATAAG
AAGGTACTTAAATAGTTGCTTTCAATTGATCTAATCGCCATTGGCACCATGAAATAAAGG
CTAATTCGTCAATCTTTGGAATGCCATAGGTTCTAGCATACGTTAACTTTTGAGTGGTGA
GTAGTTGATCATAGGGTTTACTAATAATACCAACCACAAGGATATCGACTTTTTTGTCAA
TCCCGTTGACAGGCTTT
SEQ ID 18 Position 2967081..2968319

AAATACGCAAAAGAACCCGACGAGAGTTAAGTCTCATCGGGTTCTCAGTCGTGGATGAAT
TAGAAGCATTGTTAGCTGCATAACCTTCAACATAGGATCAATAGCTGGTTAGATGGTCAT
CTCTCAGACTGTTTGCACCAGATCCAGGCAAACGTGTTTATATCCTTGGTCATACTCAGG
ATAGATGGGCATTGTGAGTGCAACAGGACTTAGTTGCTTGTATGCTAGGCAATGTTGGCC
TTGATACAGAGGATCACTCTGTTTCTGATCTGGATGATAACCTCGTTTTTCCATTTCAGG
ACGCTGTCCCAATAATTGTTGCCCTGGGAGTTTGTCGAATTAAGTCTTGGTGCCACAAAC
ACACGCTACGGCTTCTTTTCTTGCAAACTGATCTTAGTATTTAGGGCAGTTGCATGATTA
CGGAATTGAACCATTTTATAATGAATCGTCTTTTCTATAAGTCTATAGAAAGTGCAGGTA
ATGGCATTTTCTCCAGATCGGATTGTCTAATCAATTTAATTGATTTTTTTGGTGTGTTTG
ATTATATTGCTTTTGCAAAGGTACAATATACCTTTTCTCTGCTGCCTTGCGAGCAGCGAT
GGCATCCTCCATATGAACATATACACGATTTAGGACAAGATGGCCTTGAAAGTACAGCCT
TGCGACCCACTTTTGAGCAGTTTTATCCCAACTAACTCCGATAACGCCAGATTTGTTATT
GGACCGTTTAAGTGTAGAAGCAACTAAATTTGTTCGATTAATTAGTTGAAAATTCTTGGG
ATTGCCCATGTATTTTTTAGTCTGGTTATTAGCTTTTATGTTTGATCTGCTTATTTCTGA
TCGCAAACACCCACAACTGCGAGTGAGTCCTTTTTGAAGTCGTTGACTGTCAACGATACA
TTTATTTCCACATTGACATTGACAGAGCCAAAGCGCGTTTCCATTAGAACTGCTTCCAAA
AAAACTAATGACTGTGAGTCGTCCAAACGTTTGATTGGCCAAATCGATACGCTTTTGCAT
ACTAATCTCCCCGTTTGATAAGAAGGTACTTAAAGAGTTGTTTTCAATTGATCTAGTCGC
CATTGGCACCATGAAATAAAGGCTAATTCGTCAATCTTTGGAATGCCATAGGTTCTAGCA
TACGTTAACTTTTGAGTGGTAAGTAGTTGATCATAGGGTTTGCTAATAATACCAACCACA
AGGATATCGACATTTTTGTCAATCCCGTTGACAGGTTTT

### Cited Literature

Cahenzli, J., Koller, Y., Wyss, M., Geuking, M.B., and McCoy, K.D. (2013). Intestinal microbial diversity during early-life colonization shapes long-term IgE levels. Cell host & microbe 14, 559-570.
Chow, J., Tang, H., and Mazmanian, S.K. (2011). Pathobionts of the gastrointestinal microbiota and inflammatory disease. Curr Opin Immunol 23, 473-480.
Hill, D.A., Siracusa, M.C., Abt, M.C., Kim, B.S., Kobuley, D., Kubo, M., Kambayashi, T., Larosa, D.F., Renner, E.D., Orange, J.S., et al. (2012). Commensal bacteria-derived signals regulate basophil hematopoiesis and allergic inflammation. Nat. Med. 18, 538-546.
Hooper, L.V., Littman, D.R., and Macpherson, A.J. (2012). Interactions between the microbiota and the immune system. Science 336, 1268-1273.
Iovieno, A., Lambiase, A., Sacchetti, M., Stampachiacchiere, B., Micera, A., and Bonini, S. (2008). Preliminary evidence of the efficacy of probiotic eye-drop treatment in patients with vernal keratoconjunctivitis. Graefes Arch Clin Exp Ophthalmol 246, 435-441.
Khoruts, A., Dicksved, J., Jansson, J.K., and Sadowsky, M.J. (2010). Changes in the composition of the human fecal microbiome after bacteriotherapy for recurrent Clostridium difficile-associated diarrhea. J Clin Gastroenterol 44, 354-360.
Klaenhammer, T.R., Kleerebezem, M., Kopp, M.V., and Rescigno, M. (2012). The impact of probiotics and prebiotics on the immune system. Nature reviews. Immunology 12, 728-734.
Li, M., Wang, B., Zhang, M., Rantalainen, M., Wang, S., Zhou, H., Zhang, Y., Shen, J., Pang, X., Wei, H., et al. (2008). Symbiotic gut microbes modulate human metabolic phenotypes. Proceedings of the National Academy of Sciences of the United States of America 105, 2117-2122.
Mileti, E., Matteoli, G., Iliev, I.D., and Rescigno, M. (2009). Comparison of the immunomodulatory properties of three probiotic strains of Lactobacilli using complex culture systems: prediction for in vivo efficacy. PLoS One 4, e7056.
Mueller, S., Saunier, K., Hanisch, C., Norin, E., Alm, L., Midtvedt, T., Cresci, A., Silvi, S., Orpianesi, C., Verdenelli, M.C., et al. (2006). Differences in fecal microbiota in different European study populations in relation to age, gender, and country: a cross-sectional study. Applied and environmental microbiology 72, 1027-1033.
Qin, J., Li, R., Raes, J., Arumugam, M., Burgdorf, K.S., Manichanh, C., Nielsen, T., Pons, N., Levenez, F., Yamada, T., et al. (2010). A human gut microbial gene catalogue established by metagenomic sequencing. Nature 464, 59-65.
Reid, G., Younes, J.A., Van der Mei, H.C., Gloor, G.B., Knight, R., and Busscher, H.J. (2011). Microbiota restoration: natural and supplemented recovery of human microbial communities. Nat Rev Microbiol 9, 27-38.
Robles Alonso, V., and Guarner, F. (2013). Linking the gut microbiota to human health. The British journal of nutrition 109 Suppl 2, S21-26.
Swiatczak, B., Rescigno, M., and Cohen, I.R. (2011). Systemic features of immune recognition in the gut. Microbes Infect 13, 983-991.
Tsilingiri, K., Barbosa, T., Penna, G., Caprioli, F., Sonzogni, A., Viale, G., and Rescigno, M. (2012). Probiotic and postbiotic activity in health and disease: comparison on a novel polarised ex-vivo organ culture model. Gut 61, 1007-1015.
Tsilingiri, K., and Rescigno, M. (2013). Postbiotics: what else? Beneficial microbes 4, 101-107.
Yatsunenko, T., Rey, F.E., Manary, M.J., Trehan, I., Dominguez-Bello, M.G., Contreras, M., Magris, M., Hidalgo, G., Baldassano, R.N., Anokhin, A.P., et al. (2012). Human gut microbiome viewed across age and geography. Nature 486, 222-227.

## Claims

1. A method for obtaining a fermented product of Lactobacillus casei or paracasei species, said species being **characterized by** comprising in their DNA genome DNA sequences identical to SEQ ID No 2 to 5, **characterized by**:
a) growing an inoculum of Lactobacillus strain as defined above in a suitable culture medium, at a temperature ranging from 4 to 40°C, to have a biomass and allowing fermentation of said biomass to proceed for 12 to 36 hours, preferably for about 24 hours, to get a fermented biomass, and
b) centrifuging said fermented biomass to get a pellet fermented biomass and a first fermented product;
c) incubating said pellet fermented biomass into a minimum solution, preferably comprising a lactate salt, and allowing further fermentation for 12 to 36 hours, preferably for about 24 hours, to get a further fermented biomass;
d) separating said further fermented biomass from a second fermented product by centrifugation.

2. The method according to claim 1 wherein the Lactobacillus species is Lactobacillus paracasei.

3. The method according to claim 2 wherein the Lactobacillus paracasei is a strain **characterized by** comprising in its DNA genome DNA sequences essentially identical to SEQ ID No 6 to 18.

4. The method according to any of previous claims wherein the Lactobacillus paracasei is the strain deposited according to Budapest Treaty with no. CNCM I-5220.

5. A composition comprising a fermented product of Lactobacillus casei or paracasei species, said species being **characterized by** comprising in their DNA genome DNA sequences identical to SEQ ID No 2 to 5, proper diluents and/or excipients, obtainable by the method of any one of claims 1-4.

6. The composition of claim 5 further comprising adjuvants and/or other therapeutic agents.

7. The composition according claim 5 or 6 in the form of an eye-drop formulation.

8. The composition according to any of claims 5-7 wherein the fermented product is present at 2-40 % volume, preferably 10-25% vol, more preferably 25% vol.

9. The composition according to any of claims 5-8 for use in the treatment of eye inflammation syndromes, preferably of conjunctivitis, in particular of Vernal Keratoconjunctivitis (VKC), in human and veterinary medicine.

## Patentansprüche

1. Verfahren zur Gewinnung eines fermentierten Produkts der Spezies Lactobacillus casei oder paracasei, wobei die Spezies **dadurch gekennzeichnet sind, dass** sie in ihrem DNA-Genom DNA-Sequenzen umfassen, die identisch mit SEQ ID Nr. 2 bis 5 sind, **gekennzeichnet durch**:
a) Züchten eines Inokulums eines wie oben definierten Lactobacillus-Stammes in einem geeigneten Kulturmedium bei einer Temperatur im Bereich von 4 bis 40 °C, um eine Biomasse zu erhalten, und Ermöglichen einer Fermentierung der Biomasse während 12 bis 36 Stunden, vorzugsweise während etwa 24 Stunden, um eine fermentierte Biomasse zu erhalten, und
b) Zentrifugieren der fermentierten Biomasse, um eine pelletierte fermentierte Biomasse und ein erstes fermentiertes Produkt zu erhalten;
c) Inkubieren der pelletierten fermentierten Biomasse in einer Minimallösung, die vorzugsweise ein Laktatsalz enthält, und Ermöglichen einer weiteren Fermentierung während 12 bis 36 Stunden, vorzugsweise währen etwa 24 Stunden, um eine weiter fermentierte Biomasse zu erhalten;
d) Abtrennen der weiter fermentierten Biomasse von einem zweiten fermentierten Produkt durch Zentrifugation.

2. Verfahren nach Anspruch 1, wobei die Lactobacillus-Spezies Lactobacillus paracasei ist.

3. Verfahren nach Anspruch 2, wobei der Lactobacillus paracasei ein Stamm ist, der **dadurch gekennzeichnet ist, dass** er in seinem DNA-Genom DNA-Sequenzen umfasst, die im Wesentlichen identisch mit SEQ ID Nr. 6 bis 18 sind.

4. Verfahren nach einem der vorhergehenden Ansprüche, wobei der Lactobacillus paracasei der Stamm ist, der nach dem Budapester Vertrag mit der Nr. CNCM 1-5220 hinterlegt ist.

5. Zusammensetzung, umfassend ein fermentiertes Produkt der Spezies Lactobacillus casei oder paracasei, wobei die Spezies **dadurch gekennzeichnet sind, dass** sie in ihrem DNA-Genom DNA-Sequenzen umfassen, die identisch mit SEQ ID Nr. 2 bis 5 sind, geeignete Verdünnungsmittel und/oder Hilfsstoffe, erhältlich durch das Verfahren nach einem der Ansprüche 1 bis 4.

6. Zusammensetzung nach Anspruch 5, ferner umfassend Adjuvantien und/oder andere therapeutische Mittel.

7. Zusammensetzung nach Anspruch 5 oder 6 in Form einer Augentropfenformulierung.

8. Zusammensetzung nach einem der Ansprüche 5 bis 7, wobei das fermentierte Produkt mit 2 bis 40 Vol.-%, vorzugsweise 10 bis 25 Vol.-%, stärker bevorzugt 25 Vol.-% vorhanden ist.

9. Zusammensetzung nach einem der Ansprüche 5 bis 8 zur Verwendung bei der Behandlung von Augenentzündungssyndromen, vorzugsweise von Konjunktivitis, insbesondere von vernaler Keratokonjunktivitis (VKC), in der Human- und Veterinärmedizin.

## Revendications

1. Procédé d'obtention d'un produit fermenté de l'espèce Lactobacillus casei ou paracasei, ladite espèce étant **caractérisée en ce qu'**elle comprend dans son génome d'ADN des séquences d'ADN essentiellement identiques aux SEQ ID n° 2 à 5, **caractérisé par** :
a) la croissance d'un inoculum de la souche de Lactobacillus telle que définie dans un milieu de culture approprié, à une température comprise dans la plage allant de 4 à 40 °C, pour obtenir une biomasse et le fait de laisser la fermentation de ladite biomasse se dérouler pendant 12 à 36 heures, de préférence pendant environ 24 heures, pour obtenir une biomasse fermentée, et
b) la centrifugation de ladite biomasse fermentée pour obtenir une biomasse fermentée en granulés et un premier produit fermenté ;
c) l'incubation de ladite biomasse fermentée en granulés dans une solution minimale, comprenant de préférence un sel de lactate, et le fait de permettre une fermentation supplémentaire pendant 12 à 36 heures, de préférence pendant environ 24 heures, pour obtenir une biomasse fermentée supplémentaire ;
d) la séparation de ladite biomasse fermentée supplémentaire d'un second produit fermenté par centrifugation.

2. Procédé selon la revendication 1 dans lequel l'espèce Lactobacillus est Lactobacillus paracasei.

3. Procédé selon la revendication 2 dans lequel Lactobacillus paracasei est une souche **caractérisée en ce qu'**elle comprend dans son génome d'ADN des séquences d'ADN essentiellement identiques aux SEQ ID n° 6 à 18.

4. Procédé selon l'une quelconque des revendications précédentes dans lequel Lactobacillus paracasei est la souche déposée conformément au traité de Budapest sous le numéro CNCM 1-5220.

5. Composition comprenant un produit fermenté de l'espèce Lactobacillus casei ou paracasei, ladite espèce étant **caractérisée en ce qu'**elle comprend dans son génome d'ADN des séquences d'ADN identiques aux SEQ ID n° 2 à 5, des diluants et/ou des excipients appropriés, pouvant être obtenue par le procédé selon l'une quelconque des revendications 1 à 4.

6. Composition selon la revendication 5 comprenant en outre des adjuvants et/ou d'autres agents thérapeutiques.

7. Composition selon la revendication 5 ou 6 sous la forme d'une formulation de collyre.

8. Composition selon l'une quelconque des revendications 5 à 7 dans laquelle le produit fermenté est présent à hauteur de 2 à 40 % en volume, de préférence de 10 à 25 % en volume, plus préférablement de 25 % en volume.

9. Composition selon l'une quelconque des revendications 5 à 8 destinée à être utilisée dans le traitement des syndromes d'inflammation oculaire, de préférence de la conjonctivite, en particulier de la kératoconjonctivite vernale (VKC), en médecine humaine et vétérinaire.
